# EUROPEAN PATENT APPLICATION

(11) **EP 4 413 987 A1**
(43) Date of publication of application: **14.08.2024**
(21) Application number: 22877951.8
(22) Date of filing: 08.10.2022
(51) Int. Cl.: A61K 31/513, A61K 47/10, A61K 47/14, A61K 9/20, A61K 9/48

(54) **ANTIFIBROTIC COMPOSITION**

(30) Priority: 09.10.2021 CN 202111177939
(71) Applicant: Sunshine Lake Pharma Co., Ltd., Dongguan, Guangdong 523000 (CN)
(72) Inventor: YING, Xia, Dongguan, Guangdong 523871 (CN); LI, Jianze, Dongguan, Guangdong 523871 (CN); GUO, Zhen, Dongguan, Guangdong 523871 (CN); CHEN, Yina, Dongguan, Guangdong 523871 (CN); ZHENG, Yanyan, Dongguan, Guangdong 523871 (CN); ZHANG, Yingjun, Dongguan, Guangdong 523871 (CN)
(74) Representative: Hoffmann Eitle
(86) International application number: PCT/CN2022/123811
(87) International publication number: WO 2023/056935

(57) **Abstract**

An antifibrotic composition, comprising 3-(4-(dihexylamino)-3-fluorophenyl)-2,6-dimethylpyrimidin-4(3*H*)-one or a pharmaceutically acceptable salt thereof and an antioxidant. The composition has the advantages of easy storage, stable and controllable quality, high bioavailability, etc. Moreover, the composition has a high efficacy and a high safety, and the compliance of a patient taking the composition is good.

## Description

### FIELD OF THE INVENTION

The invention belongs to the field of medicines, and in particular relates to an antifibrotic composition.

### BACKGROUND ART

Fibrosis can occur in many organs, and the main pathological changes are the increase of fibrous connective tissue and the reduction of parenchymal cells in organ tissues. Continuous progress can lead to organ function decline and eventual death. It is seen in end-stage liver disease, kidney disease, idiopathic pulmonary fibrosis (IPF) and heart failure, etc., which seriously threatens human health and life. IPF is considered to be the most common and severe form of the disease, with a median survival of approximately three years, and the exact mechanisms driving fibrosis are currently unknown. Pirfenidone is the first anti-IPF drug developed by Shionogi in Japan and approved by Japan in 2008 and the European Union in 2011.

Patent application WO 2014012360 A1 discloses a substituted pyrimidinone compound, specifically the compound 3-(4-(dihexylamino)-3-fluorophenyl)-2,6-dimethylpyrimidin-4(3*H*)-one (the compound having formula (I)), which is more active than pirfenidone and has good pharmacokinetic properties and safety at the same time. Patent application WO 2018019166 A1 discloses salts of the compound having formula (I), such as hydrochloride, etc., wherein the hydrochloride has good stability and biological properties. However, the studies of the aforementioned patent applications are still in the early stage of preclinical research. In order to facilitate the development of clinical research, it is necessary to further study the properties of the drug and find a drug form suitable for human consumption.

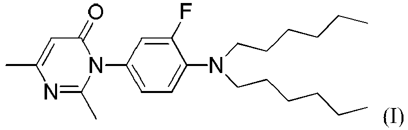

### SUMMARY

The inventors of the present application, on the basis of the self-developed new drug (the compound having formula (I) and pharmaceutically acceptable salts thereof), found through initial research that the compound having formula (I) or its pharmaceutically acceptable salt such as hydrochloride has good stability, and no impurities will be produced or the content of impurities will not increase after long-term storage. However, in the process of developing pharmaceutical formulations, the inventors found that the stability of the pharmaceutical formulation unexpectedly became very poor when the compound having formula (I) or its pharmaceutically acceptable salt such as hydrochloride was made into pharmaceutical formulations by conventional methods. In order to overcome the problem of poor stability of pharmaceutical formulations, the inventors tried to screen and proportion various pharmaceutical excipients, and found that when the compound having formula (I) or its pharmaceutically acceptable salt such as hydrochloride is combined with an antioxidant, the stability of the pharmaceutical formulation can be maintained, especially when the compound or its pharmaceutically acceptable salt such as hydrochloride is combined with an antioxidant that reacts with free radicals, the stability of the pharmaceutical formulation is better.

Based on the above findings, the present invention provides a composition with anti-fibrosis effect. The composition of the present invention contains 3-(4-(dihexylamino)-3-fluorophenyl)-2,6-dimethylpyrimidin-4(3*H*)-one (the compound having formula (I)) or its pharmaceutically acceptable salt (such as hydrochloride) as an active ingredient, which is combined with an antioxidant to overcome the problem of reduced stability of the composition.

In one aspect, the invention provides a composition. In some embodiments, the composition comprises 3-(4-(dihexylamino)-3-fluorophenyl)-2,6-dimethylpyrimidin-4(3*H*)-one (the compound having formula (I)) or a pharmaceutically acceptable salt thereof and an antioxidant. The composition contains 3-(4-(dihexylamino)-3-fluorophenyl)-2,6-dimethylpyrimidin-4(3*H*)-one or a pharmaceutically acceptable salt thereof as an active ingredient. The composition of the invention can effectively treat fibrotic diseases such as idiopathic pulmonary fibrosis and the like, and has high safety.

In some embodiments, the above composition may further include at least one of the following additional technical features:

In some embodiments, the pharmaceutically acceptable salt of the compound having formula (I) of the present invention is hydrochloride thereof.

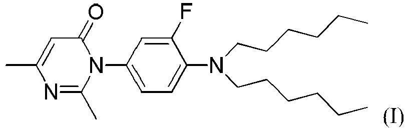

In some embodiments, the antioxidant of the present invention includes but is not limited to an antioxidant reactive with free radicals and/or an antioxidant synergist.

In some embodiments, the antioxidant of the present invention is an antioxidant reactive with free radicals.

In some embodiments, the antioxidant of the present invention includes but is not limited to butylated hydroxyanisole, butylated hydroxytoluene, propyl gallate or any combination thereof.

In some embodiments, the composition of the present invention is an oral formulation.

In some embodiments, the composition of the present invention is an oral solid formulation.

In some embodiments, the oral solid formulation of the present invention is a capsule or a tablet.

In some embodiments, the composition of the present invention is a capsule or a tablet.

In some embodiments, the composition disclosed herein further includes pharmaceutically acceptable excipients.

In some embodiments, the excipient includes at least one selected from a filler, a disintegrant, a binder, an anti-sticking agent and a lubricant.

In some embodiments, the filler of the present invention can be selected from at least one of mannitol, pregelatinized starch, microcrystalline cellulose, lactose, starch, calcium hydrogen phosphate, sorbitol, sucrose, lactitol and maltose.

In some embodiments, the disintegrant of the present invention can be selected from at least one of crospovidone, carboxymethyl starch sodium, croscarmellose sodium and low-substituted hydroxypropyl cellulose.

In some embodiments, the anti-sticking agent of the present invention can be selected from at least one of colloidal silicon dioxide and talc powder.

In some embodiments, the lubricant of the present invention can be selected from at least one of magnesium stearate, sodium stearyl fumarate, stearic acid, calcium stearate and glyceryl behenate.

In some embodiments, the binder of the present invention can be selected from at least one of povidone, hydroxypropyl methylcellulose (HPMC), hydroxypropyl cellulose, polyethylene glycol, ethylcellulose and methylcellulose.

In some embodiments, in the composition of the present invention, the content of 3-(4-(dihexylamino)-3-fluorophenyl)-2,6-dimethylpyrimidin-4(3*H*)-one hydrochloride is 5.00-73.00 parts by weight (for example: 5.00 parts by weight, 6.00 parts by weight, 7.00 parts by weight, 8.00 parts by weight, 9.00 parts by weight, 11.00 parts by weight, 12.00 parts by weight, 12.12 parts by weight, 13.00 parts by weight, 20.00 parts by weight, 21.00 parts by weight, 21.82 parts by weight , 22.00 parts by weight, 25.00 parts by weight, 28.00 parts by weight, 31.00 parts by weight, 34.00 parts by weight, 34.09 parts by weight, 35.00 parts by weight, 58.00 parts by weight, 60.00 parts by weight, 65.00 parts by weight, 70.00 parts by weight, 73.00 parts by weight or a value in the range between any two point values).

In some embodiments, the content of 3-(4-(dihexylamino)-3-fluorophenyl)-2,6-dimethylpyrimidin-4(3*H*)-one hydrochloride of the present invention is about 6.00-58.00 parts by weight.

In some embodiments, the content of 3-(4-(dihexylamino)-3-fluorophenyl)-2,6-dimethylpyrimidin-4(3*H*)-one hydrochloride is about 9.00- 58.00 or 11.00-58.00 parts by weight. In some embodiments, the content of 3-(4-(dihexylamino)-3-fluorophenyl)-2,6-dimethylpyrimidin-4(3*H*)-one hydrochloride is about 6.00-22.00 parts by weight.

In some embodiments, the content of 3-(4-(dihexylamino)-3-fluorophenyl)-2,6-dimethylpyrimidin-4(3*H*)-one hydrochloride of the present invention is 9.00- 35.00 or 12.00-35.00 parts by weight.

Preferably, the content of 3-(4-(dihexylamino)-3-fluorophenyl)-2,6-dimethylpyrimidin-4(3*H*)-one hydrochloride of the present invention is 21.00-35.00 parts by weight. Or

Preferably, the content of 3-(4-(dihexylamino)-3-fluorophenyl)-2,6-dimethylpyrimidin-4(3*H*)-one hydrochloride of the present invention is 9.00-22.00 or 12.00-22.00 parts by weight.

In some embodiments, the content of the antioxidant of the present invention is 0.01-0.40 parts by weight (for example: 0.01 parts by weight, 0.02 parts by weight, 0.03 parts by weight, 0.04 parts by weight, 0.05 parts by weight, 0.06 parts by weight, 0.07 parts by weight, 0.08 parts by weight, 0.09 parts by weight, 0.10 parts by weight, 0.15 parts by weight, 0.20 parts by weight, 0.25 parts by weight, 0.30 parts by weight, 0.40 parts by weight or a value in the range between any two point values).

In some embodiments, the content of the antioxidant of the present invention is 0.02-0.40, 0.01-0.30, 0.01-0.20, 0.02-0.30, 0.03-0.30, 0.03-0.20, 0.03-0.10, 0.05-0.20, 0.08~ 0.30, 0.08-0.20, 0.08-0.10 or 0.04-0.30 parts by weight.

In some embodiments, the content of the antioxidant of the present invention is 0.03-0.08 parts by weight. Preferably, the content of the antioxidant of the present invention is 0.03-0.05 parts by weight, 0.04-0.05 parts by weight or 0.05-0.08 parts by weight.

In some embodiments, the content of the filler of the present invention is 22.00-91.00 parts by weight (for example: 22.00 parts by weight, 22.50 parts by weight, 25.00 parts by weight, 29.00 parts by weight, 29.50 parts by weight, 30.00 parts by weight, 35.00 parts by weight, 40.00 parts by weight, 45.00 parts by weight, 50.00 parts by weight, 55.00 parts by weight, 55.50 parts by weight, 60.00 parts by weight, 60.36 parts by weight, 65.00 parts by weight, 65.60 parts by weight, 65.63 parts by weight, 67.00 parts by weight, 68.10 parts by weight, 68.13 parts by weight, 69.00 parts by weight, 70.00 parts by weight, 70.50 parts by weight, 71.00 parts by weight, 73.84 parts by weight, 74.00 parts by weight, 74.34 parts by weight, 74.50 parts by weight, 74.84 parts by weight, 75.34 parts by weight, 75.84 parts by weight, 76.00 parts by weight, 77.00 parts by weight, 80.00 parts by weight, 82.00 parts by weight, 86.00 parts by weight, 90.00 parts by weight, 90.50 parts by weight, 91.00 parts by weight or a value in the range between any two point values).

In some embodiments, the content of the filler of the present invention is about 22.50~91.00 parts by weight, 29.00~82.00 parts by weight, 55.00~90.00 parts by weight, 60.00~76.00 parts by weight, 60.00~77.00 parts by weight, 60.00~69.00 parts by weight, 55.00~90.00 parts by weight parts, 65.00~77.00 parts by weight, 65.00~76.00 parts by weight or 70.00~76.00 parts by weight.

In some embodiments, the content of the anti-sticking agent of the present invention is 0.50~3.50 parts by weight (for example: 0.50 parts by weight, 1.00 parts by weight, 1.50 parts by weight, 2.00 parts by weight, 2.50 parts by weight, 3.00 parts by weight, 3.50 parts by weight parts or a value in the range between any two point values).

In some embodiments, the content of the anti-sticking agent of the present invention is 0.50~2.50 parts by weight, 0.50~1.50 parts by weight, 1.00~2.50 parts by weight or 1.00~2.00 parts by weight.

In some embodiments, the content of the disintegrant of the present invention is 0.50~16.00 parts by weight (for example: 0.50 parts by weight, 1.00 parts by weight, 1.50 parts by weight, 2.00 parts by weight, 2.50 parts by weight, 3.00 parts by weight, 3.50 parts by weight, 4.00 parts by weight, 4.50 parts by weight, 5.00 parts by weight, 6.00 parts by weight, 7.00 parts by weight, 8.00 parts by weight, 9.00 parts by weight, 10.00 parts by weight, 12.00 parts by weight, 14.00 parts by weight, 16.00 parts by weight or a value in the range between any two point values).

In some embodiments, the content of the disintegrant of the present invention is 0.05~6.00, 1.50~6.00, 2.50~5.00, 3.00~10.00, 4.00~9.00, 4.00~8.00 or 2.50~8.00 parts by weight.

In some embodiments, the content of the lubricant of the present invention is 0.50~7.00, 0.50~6.00 or 0.50~5.00 parts by weight (for example: 0.50 parts by weight, 1.00 parts by weight, 1.50 parts by weight, 2.00 parts by weight, 2.50 parts by weight, 3.00 parts by weight, 3.50 parts by weight, 4.00 parts by weight, 4.50 parts by weight, 5.00 parts by weight, 6.00 parts by weight, 7.00 parts by weight or a value in the range between any two point values).

In some embodiments, the content of the lubricant of the present invention is 1.00~7.00, 1.00~6.00, 1.00~5.00, 2.00~7.00, 2.00~6.00, 2.00~3.00, 3.00~6.00, 5.00~7.00, 3.00~4.00 or 2.00~4.00 parts by weight.

In some embodiments, the content of the binder of the present invention is 0~3.50 parts by weight (for example: 0.01 parts by weight, 0.02 parts by weight, 0.03 parts by weight, 0.04 parts by weight, 0.05 parts by weight, 0.06 parts by weight, 0.08 parts by weight, 1.00 parts by weight, 1.50 parts by weight, 2.00 parts by weight, 2.50 parts by weight, 3.00 parts by weight, 3.50 parts by weight or a value in the range between any two point values).

In some embodiments, the content of the binder of the present invention is 0~3.00, 0~2.00, 0~2.50 or 2.00~3.00 parts by weight.

In some embodiments, the composition of the present invention is a capsule; and the capsule comprises 3-(4-(dihexylamino)-3-fluorophenyl)-2,6-dimethylpyrimidin-4(377)-one hydrochloride, an antioxidant, a filler, a disintegrant, an anti-sticking agent, a lubricant and optionally a binder.

In some embodiments, in the capsule of the present invention, the mass ratio of 3-(4-(dihexylamino)-3-fluorophenyl)-2,6-dimethylpyrimidin-4(3*H*)-one hydrochloride to the antioxidant is (5.00~73.00): (0.01~0.40).

In some embodiments, in the capsule of the present invention, the mass ratio of 3-(4-(dihexylamino)-3-fluorophenyl)-2,6-dimethylpyrimidin-4(3*H*)-one hydrochloride to the antioxidant is (21.00~35.00): (0.03~0.08).

In some embodiments, in the capsule of the present invention, the mass ratio of 3-(4-(dihexylamino)-3-fluorophenyl)-2,6-dimethylpyrimidin-4(3*H*)-one hydrochloride to the antioxidant is (21.00~35.00): (0.05~0.08).

In some embodiments, in the capsule of the present invention, the mass ratio of 3-(4-(dihexylamino)-3-fluorophenyl)-2,6-dimethylpyrimidin-4(3*H*)-one hydrochloride to the antioxidant is (21.00~22.00): (0.05~0.08).

In some embodiments, in the capsule of the present invention, the mass ratio of 3-(4-(dihexylamino)-3-fluorophenyl)-2,6-dimethylpyrimidin-4(3*H*)-one hydrochloride to the antioxidant is (34.00~35.00): (0.05~0.08).

In some embodiments, the capsule of the present invention includes: 5.00~73.00 or 21.00~35.00 parts by weight of 3-(4-(dihexylamino)-3-fluorophenyl)-2,6-dimethylpyrimidin-4(3*H*)-one hydrochloride; 0.01~0.40, 0.02~0.10 or 0.04~0.09 parts by weight of antioxidant; 22.50~90.50 parts by weight of filler; 0.50~6.00 or 2.00~6.00 parts by weight of disintegrant; 0.50~3.50 parts by weight of anti-sticking agent; 0.50~3.50 parts by weight of lubricant; and 0~2.50 parts by weight of binder.

In some embodiments, the capsule of the present invention includes: 5.00~73.00 parts by weight of 3-(4-(dihexylamino)-3-fluorophenyl)-2,6-dimethylpyrimidin-4(3*H*)-one hydrochloride; 0.01~0.40 parts by weight of antioxidant; 22.50~90.50 parts by weight of filler; 0.50~6.00 parts by weight of disintegrant; 0.50~3.50 parts by weight of anti-sticking agent; 0.50~3.50 parts by weight of lubricant; and 0~2.50 parts by weight of binder.

In some embodiments, the capsule of the present invention includes: 21.00~35.00 parts by weight of 3-(4-(dihexylamino)-3-fluorophenyl)-2,6-dimethylpyrimidin-4(3*H*)-one hydrochloride; 0.02~0.10 parts by weight of antioxidant; 22.50~90.50 parts by weight of filler; 2.00~6.00 parts by weight of disintegrant; 0.50~3.50 parts by weight of anti-sticking agent; 0.50~3.50 parts by weight of lubricant; and 0~2.50 parts by weight of binder.

In some embodiments, the capsule of the present invention includes: 21.00~35.00 parts by weight of 3-(4-(dihexylamino)-3-fluorophenyl)-2,6-dimethylpyrimidin-4(377)-one hydrochloride; 0.05~0.08 parts by weight of antioxidant; 60.00~70.00 parts by weight of filler; 2.00~6.00 parts by weight of disintegrant; 0.50~3.00 parts by weight of anti-sticking agent; 1.50~3.50 parts by weight of lubricant; and 0~2.50 parts by weight of binder.

In some embodiments, in the capsule of the present invention, the filler is optionally selected from at least one of mannitol, pregelatinized starch, microcrystalline cellulose, lactose, starch, calcium hydrogen phosphate, sorbitol, sucrose, lactitol and maltose.

In some embodiments, in the capsule of the present invention, the disintegrant is optionally selected from at least one of crospovidone, carboxymethyl starch sodium, croscarmellose sodium and low-substituted hydroxypropyl cellulose.

In some embodiments, in the capsule of the present invention, the anti-sticking agent is optionally selected from at least one of colloidal silicon dioxide and talc powder.

In some embodiments, in the capsule of the present invention, the lubricant is optionally selected from at least one of magnesium stearate, sodium stearyl fumarate, stearic acid, calcium stearate and glyceryl behenate.

In some embodiments, in the capsule of the present invention, the binder is optionally selected from at least one of povidone, hydroxypropyl methylcellulose, hydroxypropyl cellulose, polyethylene glycol, ethylcellulose and methylcellulose.

In some embodiments, in the capsule of the present invention, the antioxidant includes at least one selected from butylated hydroxyanisole, butylated hydroxytoluene and propyl gallate.

In some embodiments, in the capsule of the present invention, the filler includes at least one selected from mannitol, microcrystalline cellulose and pregelatinized starch, the disintegrant is crospovidone or carboxymethyl starch sodium, the anti-sticking agent is colloidal silicon dioxide, and the lubricant is magnesium stearate.

In some embodiments, the capsule of the present invention includes: 13.00~58.00 or 34.00~34.50 parts by weight of 3-(4-(dihexylamino)-3-fluorophenyl)-2,6-dimethylpyrimidin-4(3*H*)-one hydrochloride; 20.00~55.00 or 40.00~41.00 parts by weight of mannitol; 9.00~28.00 or 20.00~21.00 parts by weight of pregelatinized starch; 1.50~3.50 or 2.00~3.00 parts by weight of crospovidone; 0.50~2.50 or 0.50~ 1.50 parts by weight of colloidal silicon dioxide; and 0.50~3.00 or 1.50~2.50 parts by weight of magnesium stearate; and at least one selected from the following: 0.03~0.20 or 0.04~0.06 parts by weight of butylated hydroxyanisole, and 0.03~0.10 or 0.04~0.06 parts by weight of butylated hydroxytoluene.

In some embodiments, the capsule of the present invention includes: 13.00~58.00 parts by weight of 3-(4-(dihexylamino)-3-fluorophenyl)-2,6-dimethylpyrimidin-4(3*H*)-one hydrochloride; 20.00~55.00 parts by weight of mannitol; 9.00~28.00 parts by weight of pregelatinized starch; 1.50~3.50 parts by weight of crospovidone; 0.50~2.50 parts by weight of colloidal silicon dioxide; 0.50~3.00 parts by weight of magnesium stearate; and 0.04~0.06 parts by weight of butylated hydroxytoluene.

In some embodiments, the capsule of the present invention includes: 34.00~34.50 parts by weight of 3-(4-(dihexylamino)-3-fluorophenyl)-2,6-dimethylpyrimidin-4(3*H*)-one hydrochloride; 40.00~41.00 parts by weight of mannitol; 20.00~21.00 parts by weight of pregelatinized starch; 2.00~3.00 parts by weight of crospovidone; 0.50~1.50 parts by weight of colloidal silicon dioxide; and 1.50~2.50 parts by weight of magnesium stearate; and at least one selected from the following: 0.04~0.06 parts by weight of butylated hydroxyanisole, and 0.04~0.06 parts by weight of butylated hydroxytoluene.

In some embodiments, the capsule of the present invention includes: 13.00~58.00 parts by weight of 3-(4-(dihexylamino)-3-fluorophenyl)-2,6-dimethylpyrimidin-4(3*H*)-one hydrochloride; 20.00~55.00 parts by weight of mannitol; 9.00~28.00 parts by weight of pregelatinized starch; 1.50~3.50 parts by weight of crospovidone; 0.50~2.50 parts by weight of colloidal silicon dioxide; and 0.50~3.00 parts by weight of magnesium stearate; and at least one selected from the following: 0.03~0.20 parts by weight of butylated hydroxyanisole, and 0.03~0.10 parts by weight of butylated hydroxytoluene.

In some embodiments, the capsule of the present invention includes: about 34.00~34.50 parts by weight of 3-(4-(dihexylamino)-3-fluorophenyl)-2,6-dimethylpyrimidin-4(3*H*)-one hydrochloride; about 40.00~41.00 parts by weight of mannitol; about 20.00~21.00 parts by weight of pregelatinized starch; about 2.00~3.00 parts by weight of crospovidone; about 0.50~1.50 parts by weight of colloidal silicon dioxide; and about 1.50~2.50 parts by weight of magnesium stearate; and at least one selected from the following: about 0.04~0.06 parts by weight of butylated hydroxyanisole, and about 0.04~0.06 parts by weight of butylated hydroxytoluene.

In some embodiments, the capsule of the present invention includes: 21.00~22.00 parts by weight of 3-(4-(dihexylamino)-3-fluorophenyl)-2,6-dimethylpyrimidin-4(3*H*)-one hydrochloride; 43.00~47.00 parts by weight of microcrystalline cellulose; 20.00~23.00 or 21.00~23.00 parts by weight of pregelatinized starch; 4.00~6.00 or 4.00~5.50 parts by weight of carboxymethyl starch sodium; 0~2.00 parts by weight of hydroxypropyl methylcellulose; 2.00~3.00 parts by weight of colloidal silicon dioxide; and 2.00~3.50 or 2.50~3.50 parts by weight of magnesium stearate; and at least one selected from the following: 0.03~0.10 or 0.04~0.06 parts by weight of butylated hydroxyanisole, and 0.02~0.10 or 0.02~0.05 parts by weight of butylated hydroxytoluene.

In some embodiments, the capsule of the present invention includes: 21.00~22.00 parts by weight of 3-(4-(dihexylamino)-3-fluorophenyl)-2,6-dimethylpyrimidin-4(3*H*)-one hydrochloride; 43.00~47.00 parts by weight of microcrystalline cellulose; 20.00~23.00 parts by weight of pregelatinized starch; 4.00~6.00 parts by weight of carboxymethyl starch sodium; 0~2.00 parts by weight of hydroxypropyl methylcellulose; 2.00~3.00 parts by weight of colloidal silicon dioxide; and 2.00~3.50 parts by weight of magnesium stearate; and at least one selected from the following: 0.03~0.10 parts by weight of butylated hydroxyanisole, and 0.02~0.10 parts by weight of butylated hydroxytoluene.

In some embodiments, the capsule of the present invention includes: 21.00~22.00 parts by weight of 3-(4-(dihexylamino)-3-fluorophenyl)-2,6-dimethylpyrimidin-4(3*H*)-one hydrochloride; 43.00~47.00 parts by weight of microcrystalline cellulose; 21.00~23.00 parts by weight of pregelatinized starch; 4.00~5.50 parts by weight of carboxymethyl starch sodium; 0~2.00 parts by weight of hydroxypropyl methylcellulose; 2.00~3.00 parts by weight of colloidal silicon dioxide; and 2.50~3.50 parts by weight of magnesium stearate; and at least one selected from the following: 0.04~0.06 parts by weight of butylated hydroxyanisole, and 0.02~0.05 parts by weight of butylated hydroxytoluene.

In some embodiments, the capsule of the present invention includes: 21.82 parts by weight of 3-(4-(dihexylamino)-3-fluorophenyl)-2,6-dimethylpyrimidin-4(3*H*)-one hydrochloride; 43.00~47.00 parts by weight of microcrystalline cellulose; 22.00 parts by weight of pregelatinized starch; 4.50~5.00 parts by weight of carboxymethyl starch sodium; 0~2.00 parts by weight of hydroxypropyl methylcellulose; 2.50 parts by weight of colloidal silicon dioxide; and 3.00 parts by weight of magnesium stearate; and at least one selected from the following: 0.05 parts by weight of butylated hydroxyanisole, and 0.03 parts by weight of butylated hydroxytoluene.

In some embodiments, the composition of the present invention is a tablet; and the tablet comprises 3-(4-(dihexylamino)-3-fluorophenyl)-2,6-dimethylpyrimidin-4(3*H*)-one hydrochloride, an antioxidant, a filler, a disintegrant, an anti-sticking agent, a lubricant and optionally a binder.

In some embodiments, the above tablet may further include at least one of the following additional technical features:

In some embodiments, in the tablet of the present invention, the mass ratio of the 3-(4-(dihexylamino)-3-fluorophenyl)-2,6-dimethylpyrimidin-4(3*H*)-one hydrochloride to the antioxidant is (6.00~22.00): (0.02~0.30).

In some embodiments, in the tablet of the present invention, the mass ratio of the 3-(4-(dihexylamino)-3-fluorophenyl)-2,6-dimethylpyrimidin-4(3*H*)-one hydrochloride to the antioxidant is (9.00~22.00): (0.03~0.10).

In some embodiments, in the tablet of the present invention, the mass ratio of the 3-(4-(dihexylamino)-3-fluorophenyl)-2,6-dimethylpyrimidin-4(3*H*)-one hydrochloride to the antioxidant is (9.00~22.00): (0.03~0.08).

In some embodiments, in the tablet of the present invention, the mass ratio of the 3-(4-(dihexylamino)-3-fluorophenyl)-2,6-dimethylpyrimidin-4(3*H*)-one hydrochloride to the antioxidant is (12.00~22.00): (0.03~0.08).

In some embodiments, the mass ratio of the 3-(4-(dihexylamino)-3-fluorophenyl)-2,6-dimethylpyrimidin-4(3*H*)-one hydrochloride to the antioxidant is (9.00~13.00): (0.03~0.05).

In some embodiments, the mass ratio of the 3-(4-(dihexylamino)-3-fluorophenyl)-2,6-dimethylpyrimidin-4(3*H*)-one hydrochloride to the antioxidant is (12.00~13.00): (0.03~0.05).

In some embodiments, the mass ratio of the 3-(4-(dihexylamino)-3-fluorophenyl)-2,6-dimethylpyrimidin-4(3*H*)-one hydrochloride to the antioxidant is (21.00~22.00): (0.04~0.10).

In some embodiments, the mass ratio of the 3-(4-(dihexylamino)-3-fluorophenyl)-2,6-dimethylpyrimidin-4(3*H*)-one hydrochloride to the antioxidant is (21.00~22.00): (0.05~0.08).

In some embodiments, the tablet of the present invention includes: 6.00~22.00, 9.00~22.00, 9.00~13.00 or 12.00~22.00 parts by weight of 3-(4-(dihexylamino)-3-fluorophenyl)-2,6-dimethylpyrimidin-4(3*H*)-one hydrochloride; 0.02~0.30, 0.03~0.10, 0.04~0.08, 0.05~0.08 or 0.03~0.05 parts by weight of antioxidant; 55.00~90.00, 60.00~80.00, 60.00~78.00, 60.00~76.00, 60.00~77.00, 65.00~77.00 or 65.00~76.00 parts by weight of filler; 0~3.50 parts by weight of binder; 2.00~12.00, 3.00~10.00, 4.00~9.00, 4.00~8.00, 5.0~8.00 or 6.00~8.00 parts by weight of disintegrant; 0.50~3.00 or 1.00~2.50 parts by weight of anti-sticking agent; and 2.00~7.00, 2.50~6.00, 2.50~5.00 or 3.00~4.00 parts by weight of lubricant.

In some embodiments, the tablet of the present invention includes: 6.00~22.00 parts by weight of 3-(4-(dihexylamino)-3-fluorophenyl)-2,6-dimethylpyrimidin-4(3*H*)-one hydrochloride; 0.02~0.30 parts by weight of antioxidant; 55.00~90.00 parts by weight of filler; 0~3.50 parts by weight of binder; 2.00~12.00 parts by weight of disintegrant; 0.50~3.00 parts by weight of anti-sticking agent; and 2.50~7.00 or 2.50~6.00 parts by weight of lubricant.

In some embodiments, the tablet of the present invention includes: about 6.00~22.00 parts by weight of 3-(4-(dihexylamino)-3-fluorophenyl)-2,6-dimethylpyrimidin-4(3*H*)-one hydrochloride; about 0.02~0.10 parts by weight of antioxidant; about 55.00~90.00 parts by weight of filler; about 0~3.50 parts by weight of binder; about 2.00~12.00 parts by weight of disintegrant; about 0.50~3.00 parts by weight of anti-sticking agent; and about 2.50~6.00 or 2.50~5.00 parts by weight of lubricant.

In some embodiments, the tablet of the present invention includes: about 9.00~22.00 or 12.00~22.00 parts by weight of 3-(4-(dihexylamino)-3-fluorophenyl)-2,6-dimethylpyrimidin-4(3*H*)-one hydrochloride; about 0.02~0.10 parts by weight of antioxidant; about 60.00~80.00, 60.00~78.00, 60.00~76.00 or 60.00~77.00 parts by weight of filler; about 0~3.50 parts by weight of binder; about 3.00~10.00 parts by weight of disintegrant; about 1.00~2.50 parts by weight of anti-sticking agent; and about 2.50~7.00, 2.50~6.00 or 3.00~4.00 parts by weight of lubricant.

In some embodiments, the tablet of the present invention includes: about 9.00~22.00 or 12.00~22.00 parts by weight of 3-(4-(dihexylamino)-3-fluorophenyl)-2,6-dimethylpyrimidin-4(3*H*)-one hydrochloride; about 0.03~0.10 parts by weight of antioxidant; about 60.00~78.00, 60.00~76.00 or 60.00~77.00 parts by weight of filler; about 0~3.50 parts by weight of binder; about 3.00~10.00 parts by weight of disintegrant; about 1.00~2.50 parts by weight of anti-sticking agent; and about 2.50~6.00 or 3.00~4.00 parts by weight of lubricant.

In some embodiments, the tablet of the present invention includes: about 9.00~22.00 or 12.00~22.00 parts by weight of 3-(4-(dihexylamino)-3-fluorophenyl)-2,6-dimethylpyrimidin-4(3*H*)-one hydrochloride; about 0.04~0.08 parts by weight of antioxidant; about 65.00~76.00 or 65.00~77.00 parts by weight of filler; about 0~3.00 parts by weight of binder; about 4.00~8.00 or 5.00~8.00 parts by weight of disintegrant; about 1.00~2.50 parts by weight of anti-sticking agent; and about 2.50~6.00 or 3.00~4.00 parts by weight of lubricant.

In some embodiments, the tablet of the present invention includes: about 9.00~22.00 or 12.00~22.00 parts by weight of 3-(4-(dihexylamino)-3-fluorophenyl)-2,6-dimethylpyrimidin-4(3*H*)-one hydrochloride; about 0.03~0.05 parts by weight of antioxidant; about 65.00~76.00 or 65.00~77.00 parts by weight of filler; about 0~3.50 parts by weight of binder; about 4.00~8.00 or 5.0~8.00 parts by weight of disintegrant; about 1.00~2.50 parts by weight of anti-sticking agent; and about 2.50~6.00 or 3.00~4.00 parts by weight of lubricant.

In some embodiments, in the tablet of the present invention, the filler is optionally selected from at least one of mannitol, pregelatinized starch, microcrystalline cellulose, lactose, starch, calcium hydrogen phosphate, sorbitol, sucrose, lactitol and maltose.

In some embodiments, in the tablet of the present invention, the disintegrant is optionally selected from at least one of crospovidone, carboxymethyl starch sodium, croscarmellose sodium and low-substituted hydroxypropyl cellulose.

In some embodiments, in the tablet of the present invention, the anti-sticking agent is optionally selected from at least one of colloidal silicon dioxide and talc powder.

In some embodiments, in the tablet of the present invention, the lubricant is optionally selected from at least one of magnesium stearate, sodium stearyl fumarate, stearic acid, calcium stearate and glyceryl behenate.

In some embodiments, in the tablet of the present invention, the binder is optionally selected from at least one of povidone, hydroxypropyl methylcellulose, hydroxypropyl cellulose, polyethylene glycol, ethylcellulose and methylcellulose.

In some embodiments, in the tablet of the present invention, the antioxidant includes at least one selected from butylated hydroxyanisole, butylated hydroxytoluene and propyl gallate.

In some embodiments, in the tablet of the present invention, the filler includes at least one selected from mannitol, microcrystalline cellulose and pregelatinized starch, the binder is povidone or hydroxypropyl methylcellulose, the disintegrant is crospovidone or carboxymethyl starch sodium, the anti-sticking agent is colloidal silicon dioxide, and the lubricant is magnesium stearate or glyceryl behenate.

In some embodiments, the tablet of the present invention includes: 9.00~13.00, 11.00~13.00 or 12.00~13.00 parts by weight of 3-(4-(dihexylamino)-3-fluorophenyl)-2,6-dimethylpyrimidin-4(3*H*)-one hydrochloride; 9.00~10.50 or 9.00~10.00 parts by weight of pregelatinized starch; 60.00~70.00 or 62.00~67.00 or 64.00~66.00 parts by weight of microcrystalline cellulose; 1.00~4.00 or 2.00~3.00 parts by weight of povidone or hydroxypropyl methylcellulose; 4.00~12.00, 4.00~9.00, 5.00~9.00, 4.00~8.00 or 6.00~8.00 parts by weight of crospovidone; 0.50~2.50 or 1.00~2.00 parts by weight of colloidal silicon dioxide; 2.50~4.50 or 3.00~4.00 parts by weight of magnesium stearate, or 5.00~7.00 parts by weight of glyceryl behenate; and at least one selected from the following: 0.03~0.05 or 0.03~0.04 parts by weight of butylated hydroxytoluene and 0.03~0.08 or 0.03~0.05 parts by weight of butylated hydroxyanisole.

In some embodiments, the tablet of the present invention includes: 9.00~13.00 or 11.00~13.00 parts by weight of 3-(4-(dihexylamino)-3-fluorophenyl)-2,6-dimethylpyrimidin-4(3*H*)-one hydrochloride; 9.00~10.50 parts by weight of pregelatinized starch; 60.00~70.00 parts by weight of microcrystalline cellulose; 1.00~4.00 parts by weight of povidone or hydroxypropyl methylcellulose; 4.00~12.00 parts by weight of crospovidone; 0.50~2.50 parts by weight of colloidal silicon dioxide; 2.50~4.50 parts by weight of magnesium stearate, or 5.00~7.00 parts by weight of glyceryl behenate; and at least one selected from the following: 0.03~0.05 parts by weight of butylated hydroxytoluene and 0.03~0.05 parts by weight of butylated hydroxyanisole.

In some embodiments, the tablet of the present invention includes: 9.00~13.00 or 12.00~13.00 parts by weight of 3-(4-(dihexylamino)-3-fluorophenyl)-2,6-dimethylpyrimidin-4(3*H*)-one hydrochloride; 9.00~10.00 parts by weight of pregelatinized starch; 64.00~66.00 or 64.00~67.00 parts by weight of microcrystalline cellulose; 2.00~3.00 parts by weight of povidone or hydroxypropyl methylcellulose; 4.00~8.00 or 6.00~8.00 parts by weight of crospovidone; 1.00~2.00 parts by weight of colloidal silicon dioxide; 3.00~4.00 parts by weight of magnesium stearate, or 5.00~7.00 parts by weight of glyceryl behenate; and at least one selected from the following: 0.03~0.04 parts by weight of butylated hydroxytoluene and 0.03~0.05 parts by weight of butylated hydroxyanisole.

In some embodiments, the tablet of the present invention includes: 21.00~22.00 parts by weight of 3-(4-(dihexylamino)-3-fluorophenyl)-2,6-dimethylpyrimidin-4(3*H*)-one hydrochloride; 20.00~25.00 or 23.00~24.00 parts by weight of pregelatinized starch; 40.00~50.00 or 43.00~47.00 parts by weight of microcrystalline cellulose; 0~3.00 or 0~2.00 parts by weight of hydroxypropyl methylcellulose; 4.00~ 5.50 or 4.50~5.00 parts by weight of carboxymethyl starch sodium; 2.00~3.00 parts by weight of colloidal silicon dioxide; and 2.00~4.00 or 2.50~3.50 parts by weight of magnesium stearate; and at least one selected from the following: 0.04~0.08 or 0.04~0.06 parts by weight of butylated hydroxyanisole, and 0.02~0.05 or 0.02~0.04 parts by weight of butylated hydroxytoluene.

In some embodiments, the tablet of the present invention includes: 21.00~22.00 parts by weight of 3-(4-(dihexylamino)-3-fluorophenyl)-2,6-dimethylpyrimidin-4(3*H*)-one hydrochloride; 20.00~25.00 parts by weight of pregelatinized starch; 40.00~50.00 parts by weight of microcrystalline cellulose; 0~3.00 parts by weight of hydroxypropyl methylcellulose; 4.00~5.50 parts by weight of carboxymethyl starch sodium; 2.00~3.00 parts by weight of colloidal silicon dioxide; and 2.00~4.00 parts by weight of magnesium stearate; and at least one selected from the following: 0.04~0.08 parts by weight of butylated hydroxyanisole, and 0.02~0.05 parts by weight of butylated hydroxytoluene.

In some embodiments, the tablet of the present invention includes: 21.00~22.00 parts by weight of 3-(4-(dihexylamino)-3-fluorophenyl)-2,6-dimethylpyrimidin-4(3*H*)-one hydrochloride; 23.00~24.00 parts by weight of pregelatinized starch; 43.00~47.00 parts by weight of microcrystalline cellulose; 0~2.00 parts by weight of hydroxypropyl methylcellulose; 4.50~ 5.00 parts by weight of carboxymethyl starch sodium; 2.00~3.00 parts by weight of colloidal silicon dioxide; and 2.50~3.50 parts by weight of magnesium stearate; and at least one selected from the following: 0.04~0.06 parts by weight of butylated hydroxyanisole, and 0.02~0.04 parts by weight of butylated hydroxytoluene.

In some embodiments, the tablet further comprises a coating material; optionally, the coating material is a film coating premix.

In some embodiments, the weight of the coating material is 2.0-4.0% of the weight of the plain tablet of the tablet.

Additional aspects and advantages of the invention will be set forth in part in the description below, and in part will become obvious from the description, or may be learned through the practice of the invention.

### DETAILED DESCRIPTION OF EMBODIMENTS

Embodiments of the present invention are described in detail below. The embodiments described below are exemplary only for explaining the present invention and should not be construed as limiting the present invention.

It should be noted that the terms "first" and "second" are only used for descriptive purposes, and cannot be understood as indicating or implying relative importance or implicitly indicating the quantity of indicated technical features. Thus, a feature defined as "first" or "second" may explicitly or implicitly include one or more of these features. Further, in the description of the present invention, unless otherwise specified, "multiple" means two or more.

### DETAILED DESCRIPTION OF THE INVENTION

### Definitions and general terms

Before the present invention is described in more detail, it should be understood that this invention is not limited to particular embodiments described herein, as such embodiments may vary. It should also be understood that terms used herein are for the purpose of describing particular embodiments only and that terms are not intended to be limiting. Unless defined otherwise, all technical and scientific terms used herein have the same meanings as commonly understood by one of ordinary skill in the art. All publications and patents referenced herein are hereby incorporated by reference in their entirety.

In the context of the present invention, all numbers disclosed herein are approximations. The value of each number may vary by 1%, 2%, 5%, 7%, 8% or 10%. When a number with an N value is made public, any number within N +/- 1%, N +/- 2%, N +/- 3%, N +/- 5%, N +/- 7%, N +/- 8%, or N +/- 10% will be disclosed clearly, wherein "+/-" means plus or minus. Whenever a lower limit, DL, and an upper limit, DU, of a numerical range are disclosed, any numerical value within that disclosed range is expressly disclosed.

Where a range of values is provided, it is understood that, unless the context clearly dictates otherwise, the tenth of the unit of the upper and lower limits, intervening values between the upper and lower limits of that range and any other stated or intervening values in said ranges are covered by the present invention. The upper and lower limits of these smaller ranges may independently be included in the smaller ranges and are also covered in the invention, subject to any specifically excluded limitation in the stated range. Where the stated range includes one or both of the limits, ranges excluding either or both of those included limits are also included in the invention.

Certain ranges are provided herein with numerical values preceded by the term "about". The term "about" is used herein to provide literal support for an exact value as well as a value that is near or approximately preceded by that term. In determining whether a value is near or approximately a specifically stated value, the near or approximately unrecited value may be a value which, in the context in which it is provided, provides a substantial equivalent to the specifically stated value. The terms "about" or "approximately" refer to an acceptable error for a particular value, as determined by one skilled in the art, depending in part on how the value was measured or determined. In certain embodiments, the term "about" or "approximately" means within 1, 2, 3, or 4 standard deviations. In certain embodiments, the term "about" or "approximately" refers to within 30%, 25%, 20%, 15%, 10%, 9%, 8%, 7%, 6%, 5%, 4%, 3%, 2%, 1%, 0.5%, 0.1%, or 0.05% of a given value or range.

The terms "optionally", "optional" or "option" mean that the subsequently described event or circumstance can but need not occur, and that the description includes instances where the event or circumstance occurs and instances where it does not occur. For example, the composition of the present invention includes an optional binder, which means that the composition may or may not contain a binder.

The term "pharmaceutically acceptable" means that the substance or composition must be chemically and/or toxicologically compatible with the other ingredients comprising the formulation and/or with the mammal being treated therewith. Preferably, "pharmaceutically acceptable" in the present invention means approved by a regulatory agency of the Federal or a state government or listed in the U.S. Pharmacopeia or other generally recognized pharmacopeia for use in animals, and particularly in humans.

The term "pharmaceutically acceptable salt" refers to organic or inorganic salts of a compound disclosed herein. Pharmaceutically acceptable salts are well known in the art, as documented in Berge et al., describe pharmaceutically acceptable salts in detail in J. Pharmacol Sci, 1997, 66, 1-19. In some embodiments, the pharmaceutically acceptable salts described in the present invention include but are not limited to hydrochloride, benzene sulfonate, *p*-toluenesulfonate, sulfate, hydrobromide and the like. Preferably, the pharmaceutically acceptable salt of the present invention is hydrochloride.

For these and other pharmaceutically acceptable excipients or processes mentioned herein, reference is made to the extensive literature on the subject. See in particular Handbook of Pharmaceutical Excipients, 3rd edition, edited by Arthur H. Kibbe, American Pharmaceutical Association, Washington, USA and Pharmaceutical Press, London; and Lexikon der Hilfsstoffe für Pharmazie, Kosmetik and angrenzende Gebiete, edited by H.P. Fiedler, 4th edition, edited by Cantor, Aulendorf and earlier editions.

The compositions or formulations provided by the present invention can be administered to patients alone, or co-administered or combined with other active agents. The terms "co-administration" and "combination" include simultaneous or sequential administration of two or more therapeutic agents without specific time limits. In one embodiment, the agents are present in a cell or in an individual at the same time, or exert a biological or therapeutic effect at the same time. In one embodiment, each therapeutic agent is in the same composition or unit dosage form. In other embodiments, each therapeutic agent is in a different composition or unit dosage form. In certain embodiments, the first agent is administered before (e.g., before 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks), simultaneously with, or after (e.g., after 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks) the second therapeutic agent is administered.

The term "active ingredient" or "active agent" refers to a substance used in therapy (e.g., human therapy, veterinary therapy) (including prophylactic and therapeutic treatments). Active ingredients include any substance used as a medicament for the treatment, prevention, delay, alleviation or amelioration of a disease, condition or disorder.

The term "oral formulation" refers to a formulation form in which the drug is administered orally and absorbed into the blood in the gastrointestinal tract, including tablets, granules, capsules, oral solutions, and the like.

The term "solid oral formulation" refers to tablets, dispersible tablets, fast-dissolving tablets, fast-melting tablets, fast-melting tablets, mouth-dissolving tablets, mouth-melting tablets, orodispersible tablets, lyophilized units, porous tablets, conventional tablets, coated tablets, uncoated tablets, gastro-resistant tablets, effervescent tablets, soluble tablets, chewable tablets, oral lyophilizates, powders, oral powders, pills, capsules and/or granules. In some embodiments, the solid oral formulation is a capsule. In some embodiments, the solid oral formulation is a tablet.

The term "parts by weight" refers to the mass parts obtained by comparing the mass of a certain component of the composition with the mass of other components. For example, the content of the antioxidant is 0.01-0.40 parts by weight, which means that the mass parts obtained after comparing the mass of the antioxidant with other components of the composition are 0.01-0.40 parts by weight.

In the preparation of formulations, the weight data of each component may be enlarged or omitted or replaced by the same amount according to the proportion. In the present invention, according to the quantitative relationship between the molecular weight or mass of each component itself, it can be extended on the specific value or the number of decimal places or the number of digits accurate to different decimal places, including but not limited to, further analogies in accordance with the rules of rounding to the extent pharmaceutically understandable or in the case of scientific notation. For example, the content of the filler is 80.00 parts by weight, which is equivalent or interchangeable with the content of 79.95 parts by weight, 80.01 parts by weight, and 80.35 parts by weight, and other components can be deduced by analogy.

The term "antioxidant synergist" refers to that inhibits the formation of free radicals and synergize with other antioxidant systems.

The term "comprise" or "include" is an open expression, which means comprising the contents disclosed herein, but don't exclude other contents.

### Composition

In one aspect of the present invention, a composition is provided herein, which has anti-fibrosis effect and can be used for treating fibrotic diseases such as liver fibrosis, pulmonary fibrosis such as idiopathic pulmonary fibrosis and the like. In some embodiments, the composition comprises 3-(4-(dihexylamino)-3-fluorophenyl)-2,6-dimethylpyrimidin-4(3*H*)-one (the compound having formula (I)) or a pharmaceutically acceptable salt thereof and an antioxidant.

In some embodiments, the composition contains 3-(4-(dihexylamino)-3-fluorophenyl)-2,6-dimethylpyrimidin-4(3*H*)-one or a pharmaceutically acceptable salt thereof such as hydrochloride as an active ingredient. The inventors found through a large number of tests that the problem of reduced stability of the composition can be well overcome by combining the active ingredient with an antioxidant. The composition effectively reduces the formation of impurities during storage, and has the advantages of easy storage, stable and controllable quality, high bioavailability, and the like. Moreover, the inventors also found that the composition prepared by the present invention has strong drug efficacy, high safety, and good compliance of patients taking it.

In some embodiments, the pharmaceutically acceptable salt of the compound having formula (I) of the present invention is hydrochloride thereof. The composition of the present invention contains the hydrochloride of the compound having formula (I) as an active ingredient.

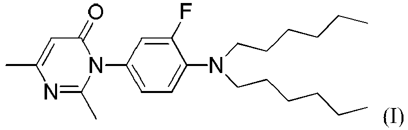

In some embodiments, the antioxidant of the present invention includes but is not limited to an antioxidant reactive with free radicals and/or an antioxidant synergist.

In some embodiments, the antioxidant of the present invention is an antioxidant reactive with free radicals; optionally, the antioxidant reactive with free radicals may be selected from at least one of butylated hydroxyanisole, butylated hydroxytoluene (also known as dibutyl hydroxytoluene) and propyl gallate. Antioxidants reactive with free radicals are a class of antioxidants capable of reacting with free radicals, which can block the chain reaction of the oxidation process. The inventors found through a large number of tests that using this type of antioxidant, the effect is better.

In some embodiments, the composition of the present invention is an oral formulation.

In some embodiments, the composition of the present invention is an oral solid formulation. The oral solid formulation made from the compound having formula (I) or a pharmaceutically acceptable salt thereof and an antioxidant has a good effect on the stability. Optionally, the oral solid formulation of the present invention is a capsule or a tablet.

In some embodiments, the composition of the present invention is a capsule or a tablet. The compound having formula (I) or a pharmaceutically acceptable salt thereof and an antioxidant are made into a capsule and/or a tablet, which has better stability effect.

In some embodiments, the composition of the present invention contains an appropriate amount of the compound having formula (I) or a pharmaceutically acceptable salt thereof. The appropriate amount is an effective dose of the compound, which is safe and effective. Preferably, in the composition, the content of the compound having formula (I) can be 4.00~70.00 parts by weight; optionally, in the composition, the content of the compound having formula (I) can be 4.50~67.00 parts by weight; optionally, in the composition, the content of the compound having formula (I) can be 20.00~32.00 parts by weight; optionally, in the composition, the content of the compound having formula (I) can be 8.00~32.00 parts by weight; optionally, in the composition, the content of the compound having formula (I) can be 11.00~32.00 parts by weight; optionally, in the composition, the content of the compound having formula (I) can be 8.00~20.00 parts by weight; optionally, in the composition, the content of the compound having formula (I) can be 11.00~20.00 parts by weight. Optionally, when the active ingredient in the composition is a pharmaceutically acceptable salt of the compound having formula (I), the content of the salt can be converted according to the ratio of the molecular weight of the free compound to the corresponding salt.

In some embodiments, the composition of the present invention contains a pharmaceutically acceptable salt of the compound having formula (I) as an active ingredient. Preferably, the composition of the present invention can contains the hydrochloride of the compound having formula (I) as an active ingredient.

In some embodiments, in the composition of the present invention, the content of the hydrochloride of the compound having formula (I) is 5.00~73.00 parts by weight; optionally, the content of the hydrochloride of the compound having formula (I) is about 6.00~58.00 parts by weight; optionally, the content of the hydrochloride of the compound having formula (I) is about 9.00~58.00 parts by weight; optionally, the content of the hydrochloride of the compound having formula (I) is about 11.00~58.00 parts by weight; optionally, the content of the hydrochloride of the compound having formula (I) is about 12.00~58.00 parts by weight; optionally, the content of the hydrochloride of the compound having formula (I) is about 13.00~58.00 parts by weight; optionally, the content of the hydrochloride of the compound having formula (I) is about 9.00~35.00 parts by weight; optionally, the content of the hydrochloride of the compound having formula (I) is about 12.00~35.00 parts by weight; optionally, the content of the hydrochloride of the compound having formula (I) is about 6.00~22.00 parts by weight; optionally, the content of the hydrochloride of the compound having formula (I) is about 9.00~22.00 parts by weight; optionally, the content of the hydrochloride of the compound having formula (I) is about 21.00~35.00 parts by weight; optionally, the content of the hydrochloride of the compound having formula (I) is about 12.0~22.0 parts by weight; optionally, the content of the hydrochloride of the compound having formula (I) is 12.12~34.09 parts by weight; optionally, the content of the hydrochloride of the compound having formula (I) is 12.12~21.82 parts by weight; optionally, the content of the hydrochloride of the compound having formula (I) is 21.82~34.09 parts by weight; optionally, the content of the hydrochloride of the compound having formula (I) is about 9, 12, 22 or 34 parts by weight; optionally, the content of the hydrochloride of the compound having formula (I) is 9.09, 12.12, 21.82 or 34.09 parts by weight.

In some embodiments, in the composition, the content of the antioxidant is 0.01~0.40 parts by weight; optionally, the content of the antioxidant is 0.02~0.40 parts by weight; optionally, the content of the antioxidant is 0.01~0.30 parts by weight; optionally, the content of the antioxidant is 0.01~0.20 parts by weight; optionally, the content of the antioxidant is 0.02~0.30 parts by weight; optionally, the content of the antioxidant is 0.03~0.30 parts by weight; optionally, the content of the antioxidant is 0.03~0.20 parts by weight; optionally, the content of the antioxidant is 0.03~0.10 parts by weight; optionally, the content of the antioxidant is 0.05~0.20 parts by weight; optionally, the content of the antioxidant is 0.08~0.30 parts by weight part; optionally, the content of the antioxidant is 0.08~0.20 parts by weight; optionally, the content of the antioxidant is 0.08~0.10 parts by weight; optionally, the content of the antioxidant is 0.04~0.30 parts by weight; optionally, the content of the antioxidant is 0.03~0.08 parts by weight; optionally, the content of the antioxidant is 0.03~0.05 parts by weight; optionally, the content of the antioxidant is 0.04~0.05 parts by weight; optionally, the content of the antioxidant is 0.05~0.08 parts by weight.

The inventors found through a large number of tests that controlling the content of the antioxidant at 0.01~0.4 parts by weight, especially 0.03~0.10 parts by weight, can effectively reduce the amount and rate of impurities generated during the storage of the composition, improve the stability of the composition, and facilitate the storage of the composition.

In some embodiments, the composition further includes pharmaceutically acceptable excipients. The stability of the composition can be further improved by adding excipients.

In some embodiments, the excipient includes at least one selected from a filler, a disintegrant, a binder, an anti-sticking agent and a lubricant. The stability of the composition can be further improved by adding the above-mentioned types of excipients.

In some embodiments, the filler of the present invention can be selected from at least one of mannitol, pregelatinized starch, microcrystalline cellulose, lactose, starch, calcium hydrogen phosphate, sorbitol, sucrose, lactitol and maltose.

In some embodiments, the disintegrant of the present invention can be selected from at least one of crospovidone, carboxymethyl starch sodium, croscarmellose sodium and low-substituted hydroxypropyl cellulose.

In some embodiments, the anti-sticking agent of the present invention can be selected from at least one of colloidal silicon dioxide and talc powder.

In some embodiments, the lubricant of the present invention can be selected from at least one of magnesium stearate, sodium stearyl fumarate, stearic acid, calcium stearate and glyceryl behenate.

In some embodiments, the binder of the present invention can be selected from at least one of povidone, hydroxypropyl methylcellulose (HPMC), hydroxypropyl cellulose, polyethylene glycol, ethylcellulose and methylcellulose.

In some embodiments, in the composition of the present invention, the content of the filler is 22.00~91.00 parts by weight. Optionally, the content of the filler is 22.50~91.00 parts by weight; optionally, the content of the filler is about 29.00~82.00 parts by weight; optionally, the content of the filler is about 55.00~90.00 parts by weight; optionally, the content of the filler is about 55.00~82.00 parts by weight; optionally, the content of the filler is about 70.00~76.00 parts by weight; optionally, the content of the filler is about 65.00~77.00 parts by weight; optionally, the content of the filler is about 65.00~76.00 parts by weight; optionally, the content of the filler is about 60.00~77.00 parts by weight; optionally, the content of the filler is 60.00~76.00 parts by weight; optionally, the content of the filler is about 60.00~69.00 parts by weight.

In some embodiments, the content of the anti-sticking agent of the present invention is 0.50~3.50 parts by weight. Optionally, the content of the anti-sticking agent is 0.50~2.50 parts by weight; optionally, the content of the anti-sticking agent is 0.50~1.50 parts by weight; optionally, the content of the anti-sticking agent is 1.00~2.50 parts by weight; optionally, the content of the anti-sticking agent is 1.00~2.00 parts by weight.

In some embodiments, the content of the disintegrant of the present invention is 0.50~16.00 parts by weight. Optionally, the content of the disintegrant is 1.50~12.00 parts by weight; optionally, the content of the disintegrant is 2.00~10.00 parts by weight; optionally, the content of the disintegrant is 3.00~10.00 parts by weight; optionally, the content of the disintegrant is 0.50~6.00 parts by weight; optionally, the content of the disintegrant is 1.50~6.00 parts by weight; optionally, the content of the disintegrant is 2.50~5.00 parts by weight; optionally, the content of the disintegrant is 4.00~9.00 parts by weight; optionally, the content of the disintegrant is 4.00~8.00 parts by weight; optionally, the content of the disintegrant is 1.50~3.50 parts by weight; optionally, the content of the disintegrant is 2.50~6.00 parts by weight; optionally, the content of the disintegrant is 2.50~8.00 parts by weight.

In some embodiments, the content of the lubricant of the present invention is 0.50~7.00 parts by weight. Optionally, the content of the lubricant is 0.50~5.00 parts by weight; optionally, the content of the lubricant is 1.00~7.00 parts by weight; optionally, the content of the lubricant is 1.00~6.00 parts by weight; optionally, the content of the lubricant is 1.00~5.00 parts by weight; optionally, the content of the lubricant is 2.00~7.00 parts by weight; optionally, the content of the lubricant is 2.00~6.00 parts by weight; optionally, the content of the lubricant is 2.00~4.00 parts by weight; optionally, the content of the lubricant is 2.00~3.00 parts by weight; optionally, the content of the lubricant is 3.00~6.00 parts by weight; optionally, the content of the lubricant is 3.00~4.00 parts by weight; optionally, the content of the lubricant is 2.00~3.50 parts by weight; optionally, the content of the lubricant is 2.00~4.00 parts by weight; optionally, the content of the lubricant is 5.00~7.00 parts by weight.

In some embodiments, the content of the binder of the present invention is 0~3.50 parts by weight. Optionally, the content of the binder is 0~3.00 parts by weight; optionally, the content of the binder is 0~2.50 parts by weight; optionally, the content of the binder is 0~2.00 parts by weight; optionally, the content of the binder is 2.00~3.00 parts by weight; optionally, the content of the binder is 2.50 parts by weight.

### Capsule

In yet another aspect of the present invention, a capsule is provided herein. In some embodiments, the capsule comprises a compound having formula (I) or a pharmaceutically acceptable salt thereof, an antioxidant, a filler, a disintegrant, an anti-sticking agent, a lubricant and an optional binder. Wherein, the "optional binder" means that the capsule may or may not contain a binder.

The inventors found through a large number of tests that the capsule prepared by using the above formula has less impurity content and good dissolution rate during storage, and can improve the stability and safety of the capsule.

In some embodiments, in the capsule of the present invention, the mass ratio of the compound having formula (I) or a pharmaceutically acceptable salt thereof to the antioxidant is about (4.00~77.00): (0.01~0.40). The inventors found through experiments that the use of the above ratio can reduce the amount and rate of impurities generated during the storage of the capsules, improve the stability of the capsules, and facilitate the storage of the capsules.

In some embodiments, in the capsule of the present invention, the active ingredient is the hydrochloride of the compound having formula (I).

In some embodiments, in the capsule of the present invention, the mass ratio of the hydrochloride of the compound having formula (I) to the antioxidant is (5.00~73.00): (0.01~0.40). The inventors found through a large number of tests that the capsules prepared within the above proportioning range have less impurity content and high stability during storage.

In some embodiments, in the capsule of the present invention, the mass ratio of the hydrochloride of the compound having formula (I) to the antioxidant is (13.00~58.00): (0.03~0.30); optionally, the mass ratio of the hydrochloride of the compound having formula (I) to the antioxidant is (21.00~35.00): (0.03~0.10); optionally, the mass ratio of the hydrochloride of the compound having formula (I) to the antioxidant is (21.00~35.00): (0.03~0.80); optionally, the mass ratio of the hydrochloride of the compound having formula (I) to the antioxidant is (21.00~35.00): (0.05~0.80); optionally, the mass ratio of the hydrochloride of the compound having formula (I) to the antioxidant is (21.00~22.00): (0.05~0.08); optionally, the mass ratio of the hydrochloride of the compound having formula (I) to the antioxidant is (34.00~35.00): (0.05~0.08).

In some embodiments, the capsule of the present invention includes: 5.00~73.00 or 21.00~35.00 parts by weight of 3-(4-(dihexylamino)-3-fluorophenyl)-2,6-dimethylpyrimidin-4(3*H*)-one hydrochloride; 0.01~0.40, 0.02~0.10 or 0.04~0.09 parts by weight of antioxidant; 22.50~90.50 parts by weight of filler; 0.50~6.00 or 2.00~6.00 parts by weight of disintegrant; 0.50~3.50 parts by weight of anti-sticking agent; 0.50~3.50 parts by weight of lubricant; and 0~2.50 parts by weight of binder.

In some embodiments, the capsule of the present invention includes: 5.00~73.00 parts by weight of the hydrochloride of the compound having formula (I); 0.01~0.40 parts by weight of antioxidant; 22.50~90.50 parts by weight of filler; 0.50~6.00 parts by weight of disintegrant; 0.50~3.50 parts by weight of anti-sticking agent; 0.50~3.50 parts by weight of lubricant; and/or 0~2.50 parts by weight of binder.

In some embodiments, in the capsule of the present invention, the antioxidant is an antioxidant reactive with free radicals; optionally, the antioxidant reactive with free radicals may be selected from at least one of butylated hydroxyanisole, butylated hydroxytoluene and propyl gallate.

The inventors found through a large number of tests that the capsule prepared by using the above formula has less impurity content and good dissolution rate during storage, and can improve the stability and safety of the capsule.

In some embodiments, in the capsule of the present invention, the filler is optionally selected from at least one of mannitol, pregelatinized starch, microcrystalline cellulose, lactose, starch, calcium hydrogen phosphate, sorbitol, sucrose, lactitol and maltose.

In some embodiments, in the capsule of the present invention, the disintegrant is optionally selected from at least one of crospovidone, carboxymethyl starch sodium, croscarmellose sodium and low-substituted hydroxypropyl cellulose.

In some embodiments, in the capsule of the present invention, the anti-sticking agent is optionally selected from at least one of colloidal silicon dioxide and talc powder.

In some embodiments, in the capsule of the present invention, the lubricant is optionally selected from at least one of magnesium stearate, sodium stearyl fumarate, stearic acid, calcium stearate and glyceryl behenate.

In some embodiments, in the capsule of the present invention, the binder is optionally selected from at least one of povidone, hydroxypropyl methylcellulose, hydroxypropyl cellulose, polyethylene glycol, ethylcellulose and methylcellulose.

In some embodiments, in the capsule of the present invention, the antioxidant is butylated hydroxyanisole, butylated hydroxytoluene and/or propyl gallate, the filler is mannitol, microcrystalline cellulose and/or pregelatinized starch, the disintegrant is crospovidone or carboxymethyl starch sodium, the anti-sticking agent is colloidal silicon dioxide, and the lubricant is magnesium stearate. The inventors found through experiments that the capsules prepared from the above-mentioned raw materials have less impurities during storage, and have the advantages of easy storage and good stability.

In some embodiments, the capsule of the present invention includes: 13.00~58.00 parts by weight of the hydrochloride of the compound having formula (I); 20.00~55.00 parts by weight of mannitol; 9.00~28.00 parts by weight of pregelatinized starch; 1.50~3.50 parts by weight of crospovidone; 0.50~1.50 parts by weight of colloidal silicon dioxide; and 0.50~2.00 parts by weight of magnesium stearate; and at least one selected from the following: 0.05~0.20 parts by weight of butylated hydroxyanisole, and 0.03~0.10 parts by weight of butylated hydroxytoluene.

In some embodiments, the capsule includes: about 34.00~34.50 parts by weight of the hydrochloride of the compound having formula (I); about 40.00~41.00 parts by weight of mannitol; about 20.00~21.00 parts by weight of pregelatinized starch; about 2.00~2.50 parts by weight of crospovidone; about 1.00~1.50 parts by weight of colloidal silicon dioxide; and about 1.50~2.00 parts by weight of magnesium stearate; and at least one selected from the following: about 0.05~0.20 parts by weight of butylated hydroxyanisole, and about 0.03~0.10 parts by weight of butylated hydroxytoluene.

In some embodiments, the capsule includes: 21.00~22.00 parts by weight of the hydrochloride of the compound having formula (I); 43.00~47.00 parts by weight of microcrystalline cellulose; 20.00~22.00 parts by weight of pregelatinized starch; 4.00~5.00 parts by weight of carboxymethyl starch sodium; 0~2.00 parts by weight of hydroxypropyl methylcellulose; 2.00~3.00 parts by weight of colloidal silicon dioxide; and 2.50~3.50 parts by weight of magnesium stearate; and at least one selected from the following: 0.05 parts by weight of butylated hydroxyanisole, and 0.03~0.05 parts by weight of butylated hydroxytoluene.

In some embodiments, the capsule includes: 21.82 parts by weight of the hydrochloride of the compound having formula (I); 43.00~47.00 parts by weight of microcrystalline cellulose; 22.00 parts by weight of pregelatinized starch; 4.50 parts by weight of carboxymethyl starch sodium; 0~2.00 parts by weight of hydroxypropyl methylcellulose; 2.50 parts by weight of colloidal silicon dioxide; and 3.00 parts by weight of magnesium stearate; and at least one selected from the following: 0.05 parts by weight of butylated hydroxyanisole, and 0.03~0.05 parts by weight of butylated hydroxytoluene.

The inventors found through a large number of tests that the capsules prepared by adopting the above formula have better stability.

In addition, those skilled in the art can understand that the features and advantages described above for the composition are also applicable to the capsule, and will not be repeated here.

### Tablet

In another aspect of the present invention, a tablet is provided herein. In some embodiments, the composition is a tablet, wherein the tablet comprises a compound having formula (I) or a pharmaceutically acceptable salt thereof, an antioxidant, a filler, a disintegrant, an anti-sticking agent, a lubricant and an optional binder.

The inventors found through a large number of tests that the tablet prepared by using the above formula has less impurity content and good dissolution rate during storage, and can improve the stability and safety of the tablet.

In some embodiments, in the tablet of the present invention, the mass ratio of the compound having formula (I) or a pharmaceutically acceptable salt thereof to the antioxidant is (6.00~22.00): (0.02~0.30). The inventors found through a large number of tests that the use of the above ratio can reduce the amount and rate of impurities generated during the storage of the tablets, improve the stability of the tablets, and facilitate the storage of the tablets.

In some embodiments, the tablet of the present invention contains the hydrochloride of the compound having formula (I) as an active ingredient, optionally, the mass ratio of the hydrochloride of the compound having formula (I) to the antioxidant is (6.00~22.00): (0.02~0.30). The inventors found through a large number of tests that the tablets prepared with the above proportions have less impurity content and high stability during storage.

In some embodiments, in the tablet of the present invention, the mass ratio of the hydrochloride of the compound having formula (I) to the antioxidant is about (9.00~22.00): (0.03~0.10); optionally, the mass ratio of the hydrochloride of the compound having formula (I) to the antioxidant is about (9.00~22.00): (0.03~0.08); optionally, the mass ratio of the hydrochloride of the compound having formula (I) to the antioxidant is about (12.00~22.00): (0.03~0.08); optionally, the mass ratio of the hydrochloride of the compound having formula (I) to the antioxidant is about (9.00~22.00): (0.03~0.05); optionally, the mass ratio of the hydrochloride of the compound having formula (I) to the antioxidant is about (9.00~13.00): (0.03~0.05); optionally, the mass ratio of the hydrochloride of the compound having formula (I) to the antioxidant is about (12.00~13.00): (0.03~0.05); optionally, the mass ratio of the hydrochloride of the compound having formula (I) to the antioxidant is about (12.00~22.00): (0.04~0.08); optionally, the mass ratio of the hydrochloride of the compound having formula (I) to the antioxidant is about (21.00~22.00): (0.04~0.10); optionally, the mass ratio of the hydrochloride of the compound having formula (I) to the antioxidant is about (21.00~22.00): (0.03~0.08); optionally, the mass ratio of the hydrochloride of the compound having formula (I) to the antioxidant is about (21.00~22.00): (0.05 ~0.08); optionally, the mass ratio of the hydrochloride of the compound having formula (I) to the antioxidant is about (21.00~22.00): (0.03~0.05); optionally, the mass ratio of the hydrochloride of the compound having formula (I) to the antioxidant is about (9.00 - 22.00): (0.04); optionally, the mass ratio of the hydrochloride of the compound having formula (I) to the antioxidant is about (12.00~22.00): (0.04).

In some embodiments, the tablet of the present invention includes: 6.00~22.00, 9.00~22.00, 9.00~13.00 or 12.00~22.00 parts by weight of 3-(4-(dihexylamino)-3-fluorophenyl)-2,6-dimethylpyrimidin-4(3*H*)-one hydrochloride; 0.02~0.30, 0.03~0.10, 0.03~0.08, 0.04~0.08, 0.05~0.08 or 0.03~0.05 parts by weight of antioxidant; 55.00~90.00, 60.00~80.00, 60.00~78.00, 60.00~76.00, 60.00~77.00, 65.00~77.00 or 65.00~76.00 parts by weight of filler; 0~3.50 parts by weight of binder; 2.00~12.00, 3.00~10.00, 4.00~9.00, 4.00~8.00, 5.0~8.00 or 6.00~8.00 parts by weight of disintegrant; 0.50~3.00 or 1.00~2.50 parts by weight of anti-sticking agent; and 2.00~7.00, 2.50~6.00, 2.50~5.00 or 3.00~4.00 parts by weight of lubricant.

In some embodiments, the tablet of the present invention includes: about 6.00~22.00 parts by weight of the hydrochloride of the compound having formula (I); about 0.02~0.10 parts by weight of antioxidant; about 55.00~90.00 parts by weight of filler; about 0~3.50 parts by weight of binder; about 2.00~12.00 parts by weight of disintegrant; about 0.50~3.00 parts by weight of anti-sticking agent; and about 2.50~7.00 parts by weight of lubricant.

In some embodiments, the tablet of the present invention includes: about 6.00~22.00 parts by weight of the hydrochloride of the compound having formula (I); about 0.02~0.30 parts by weight of antioxidant; about 55.00~90.00 parts by weight of filler; about 0~3.50 parts by weight of binder; about 2.00~12.00 parts by weight of disintegrant; about 0.50~3.00 parts by weight of anti-sticking agent; and about 2.50~7.00, 2.50~6.00 or 2.50~5.00 parts by weight of lubricant.

In some embodiments, the tablet of the present invention includes: about 9.00~22.00 or 12.00~22.00 parts by weight of 3-(4-(dihexylamino)-3-fluorophenyl)-2,6-dimethylpyrimidin-4(3*H*)-one hydrochloride; about 0.02~0.10 parts by weight of antioxidant; about 60.00~80.00, 60.00~78.00, 60.00~76.00 or 60.00~77.00 parts by weight of filler; about 0~3.50 parts by weight of binder; about 3.00~10.00 parts by weight of disintegrant; about 1.00~2.50 parts by weight of anti-sticking agent; and about 2.50~7.00, 2.50~6.00 or 3.00~4.00 parts by weight of lubricant.

In some embodiments, the tablet of the present invention includes: about 9.00~22.00 or 12.00~22.00 parts by weight of 3-(4-(dihexylamino)-3-fluorophenyl)-2,6-dimethylpyrimidin-4(3*H*)-one hydrochloride; about 0.04~0.08 parts by weight of antioxidant; about 65.00~76.00 or 65.00~77.00 parts by weight of filler; about 0~3.00 parts by weight of binder; about 4.00~8.00 or 5.00~8.00 parts by weight of disintegrant; about 1.00~2.50 parts by weight of anti-sticking agent; and about 2.50~6.00 or 3.00~4.00 parts by weight of lubricant.

In some embodiments, the antioxidant of the present invention is an antioxidant reactive with free radicals; optionally, the antioxidant reactive with free radicals may be selected from at least one of butylated hydroxyanisole, butylated hydroxytoluene and propyl gallate.

The inventors found through a large number of tests that the tablet prepared by using the above formula has less impurity content and good dissolution rate during storage, and can improve the stability and safety of the tablet.

In some embodiments, the filler of the present invention is optionally selected from at least one of mannitol, pregelatinized starch, microcrystalline cellulose, lactose, starch, calcium hydrogen phosphate, sorbitol, sucrose, lactitol and maltose.

In some embodiments, the disintegrant of the present invention is optionally selected from at least one of crospovidone, carboxymethyl starch sodium, croscarmellose sodium and low-substituted hydroxypropyl cellulose.

In some embodiments, the anti-sticking agent of the present invention is optionally selected from at least one of colloidal silicon dioxide and talc powder.

In some embodiments, the lubricant of the present invention is optionally selected from at least one of magnesium stearate, sodium stearyl fumarate, stearic acid, calcium stearate and glyceryl behenate.

In some embodiments, the binder of the present invention is optionally selected from at least one of povidone, hydroxypropyl methylcellulose, hydroxypropyl cellulose, polyethylene glycol, ethylcellulose and methylcellulose.

In some embodiments, the antioxidant of the present invention includes at least one selected from butylated hydroxyanisole, butylated hydroxytoluene and propyl gallate, the filler includes at least one selected from mannitol, microcrystalline cellulose and pregelatinized starch, the binder is povidone or hydroxypropyl methylcellulose, the disintegrant is crospovidone or carboxymethyl starch sodium, the anti-sticking agent is colloidal silicon dioxide, and the lubricant is magnesium stearate. The inventors found through a large number of tests that the tablets prepared from the above-mentioned raw materials have less impurities during storage, and have the advantages of easy storage and good stability.

In some embodiments, the tablet of the present invention includes: 9.00~13.00, 11.00~13.00 or 12.00~13.00 parts by weight of 3-(4-(dihexylamino)-3-fluorophenyl)-2,6-dimethylpyrimidin-4(3*H*)-one hydrochloride; 9.00~10.50 or 9.00~10.00 parts by weight of pregelatinized starch; 60.00~70.00 or 62.00~67.00 or 64.00~66.00 parts by weight of microcrystalline cellulose; 1.00~4.00 or 2.00~3.00 parts by weight of povidone or hydroxypropyl methylcellulose; 4.00~12.00, 4.00~9.00, 5.00~9.00, 4.00~8.00 or 6.00~8.00 parts by weight of crospovidone; 0.50~2.50 or 1.00~2.00 parts by weight of colloidal silicon dioxide; 2.50~4.50 or 3.00~4.00 parts by weight of magnesium stearate, or 5.00~7.00 parts by weight of glyceryl behenate; and at least one selected from the following: 0.03~0.05 or 0.03~0.04 parts by weight of butylated hydroxytoluene and 0.03~0.08 or 0.03~0.05 parts by weight of butylated hydroxyanisole.

In some embodiments, the tablet of the present invention includes: 9.00~13.00 or 11.00~13.00 parts by weight of the hydrochloride of the compound having formula (I); 9.00~10.50 parts by weight of pregelatinized starch; 60.00~70.00 parts by weight of microcrystalline cellulose; 1.00~4.00 parts by weight of povidone or hydroxypropyl methylcellulose; 4.00~12.00 parts by weight of crospovidone; 0.50~2.50 parts by weight of colloidal silicon dioxide; 3.50~4.50 parts by weight of magnesium stearate, or 5.00~7.00 parts by weight of glyceryl behenate; and at least one selected from the following: 0.03~0.05 parts by weight of butylated hydroxytoluene and 0.03~0.05 parts by weight of butylated hydroxyanisole.

In some embodiments, the tablet of the present invention includes: 9.00~13.00 or 12.00~13.00 parts by weight of the hydrochloride of the compound having formula (I); 9.00~10.00 parts by weight of pregelatinized starch; 64.00~67.00 or 64.00~66.00 parts by weight of microcrystalline cellulose; 2.00~3.00 parts by weight of povidone or hydroxypropyl methylcellulose; 4.00~8.00 or 6.00~8.00 parts by weight of crospovidone; 1.00~2.00 parts by weight of colloidal silicon dioxide; and 3.00~4.00 parts by weight of magnesium stearate, or 5.00~7.00 parts by weight of glyceryl behenate; and at least one selected from the following: 0.03~0.04 parts by weight of butylated hydroxytoluene and 0.03~0.05 parts by weight of butylated hydroxyanisole.

In some embodiments, the tablet of the present invention includes: 21.00~22.00 parts by weight of 3-(4-(dihexylamino)-3-fluorophenyl)-2,6-dimethylpyrimidin-4(3*H*)-one hydrochloride; 20.00~25.00 or 23.00~24.00 parts by weight of pregelatinized starch; 40.00~50.00 or 43.00~47.00 parts by weight of microcrystalline cellulose; 0~3.00 or 0~2.00 parts by weight of hydroxypropyl methylcellulose; 4.00~5.50 or 4.50~5.00 parts by weight of carboxymethyl starch sodium; 2.00~3.00 parts by weight of colloidal silicon dioxide; and 2.00~4.00 or 2.50~3.50 parts by weight of magnesium stearate; and at least one selected from the following: 0.04~0.08 or 0.04~0.06 parts by weight of butylated hydroxyanisole and 0.02~0.05 or 0.02~0.04 parts by weight of butylated hydroxytoluene.

In some embodiments, the tablet of the present invention includes: 21.00 - 22.00 parts by weight of the hydrochloride of the compound having formula (I); 20.00~25.00 parts by weight of pregelatinized starch; 40.00~50.00 parts by weight of microcrystalline cellulose; 0~3.00 parts by weight of hydroxypropyl methylcellulose; 4.00~ 5.50 parts by weight of carboxymethyl starch sodium; 2.00~3.00 parts by weight of colloidal silicon dioxide; and 2.00~4.00 parts by weight of magnesium stearate; and at least one selected from the following: 0.04~0.08 parts by weight of butylated hydroxyanisole and 0.02~0.05 parts by weight of butylated hydroxytoluene.

In some embodiments, the tablet of the present invention includes: 21.00~22.00 parts by weight of the hydrochloride of the compound having formula (I); 23.00~24.00 parts by weight of pregelatinized starch; 43.00~47.00 parts by weight of microcrystalline cellulose; 0~2.00 parts by weight of hydroxypropyl methylcellulose; 4.50~ 5.00 parts by weight of carboxymethyl starch sodium; 2.00~3.00 parts by weight of colloidal silicon dioxide; and 2.50~3.50 parts by weight of magnesium stearate; and at least one selected from the following: 0.04~0.06 parts by weight of butylated hydroxyanisole and 0.02~0.04 parts by weight of butylated hydroxytoluene. The inventors found through a large number of tests that the stability and drug efficacy of the tablet can be further improved when the above-mentioned formula ratio is used to prepare the tablets .

In some embodiments, the tablet further comprises a coating material; optionally, the weight of the coating material is 2.00-4.00% of the weight of the plain tablet of the tablet. Plain tablet refers to the total substance in the tablet that is wrapped in the coating material.

In addition, those skilled in the art can understand that the features and advantages described above for the composition are also applicable to the tablet, and will not be repeated here.

The solutions of the present invention will be explained below in conjunction with examples. Those skilled in the art will understand that the following examples are only for illustrating the present invention and should not be considered as limiting the scope of the present invention. If no specific technique or condition is indicated in the examples, it shall be carried out according to the technique or condition described in the literature in this field or according to the product specification.

The impurity content of the samples in each embodiment or comparative example of the present invention was determined with reference to high performance liquid chromatography (General Rule 0512 of Chinese Pharmacopoeia 2020 edition), specifically as follows:

Testing sample solution: the testing sample (for example, a capsule or tablet of the present invention, or the comparison sample in the comparative example of the present invention) was extracted and diluted with methanol into a solution containing about 2.5 mg of the compound having formula (I) per 1 mL (the tablet need to be disintegrated with a small amount of water before extraction), and then diluted with 60% methanol-water into a solution containing about 1 mg of the compound having formula (I) per 1 mL.

Control sample solution: an appropriate amount of the control sample (such as the hydrochloride of the compound having formula (I)) was taken and weighed accurately, then dissolved and diluted with 60% methanol-water into a solution containing about 5 µg of the compound having formula (I) per 1 mL.

Sensitivity solution: an appropriate amount of the control sample solution was weighed accurately, and quantitatively diluted with 60% methanol-water into a solution containing about 0.5 µg of the compound having formula (I) per 1 mL.

System suitability solution: an appropriate amount of the control sample (such as the hydrochloride of the compound having formula (I)) was weighed, dissolved and diluted with 60% methanol-water into a solution containing about 1 mg of the compound having formula (I) per 1 mL.

Chromatographic conditions: chromatographic column packed with octylsilane bonded silica gel (such as Phenomenex kinetex C8 100A, 4.6 × 100mm, 2.6 µm or equivalent chromatographic column) was used for gradient elution with 0.01 mol/L ammonium acetate-acetonitrile (9:1) as mobile phase A and methanol-acetonitrile (75:25) as mobile phase B according to the procedure in the table below; the detection wavelength was 266nm, and the injection volume was 10µL.

| Time (min) | Mobile phase A | Mobile phase B |
|---|---|---|
| 0 | 42 | 58 |
| 6 | 33 | 67 |
| 25 | 33 | 67 |
| 35 | 13 | 87 |
| 50 | 13 | 87 |
| 50.5 | 42 | 58 |
| 55 | 42 | 58 |

Calculation: Calculation is carried out by the method of principal component external standard plus correction factor.

Acceptance standard: if there are impurity peaks in the chromatogram of the testing sample solution, the total impurities shall not exceed 1.5%.

Taking the hydrochloride crystal form I of the compound having formula (I) disclosed in the patent application WO2018019166A1 as an example, the technical solution of the present invention is explained, that is, the composition is prepared by using the hydrochloride crystal form I of the compound having formula (I) as an active ingredient.

### Example 1

The active ingredient was prepared into capsules and the dissolution was determined.

Wherein, the preparation method is described as follows:
Method A:
   ① the active ingredient was mixed with fillers, disintegrants, anti-sticking agents, binders (if any) and antioxidants to obtain a premix; ② then the lubricants and the premix were mixed to obtain a total mixed powder; and ③ the total mixed powder was filled into suitable hollow capsule shells to obtain capsules.
Method B:
   ① the active ingredient was mixed with fillers, disintegrants, anti-sticking agents, binders (if any), lubricants and antioxidants to obtain a premix; ② the premix was subjected to dry granulation to obtain granulated granules; ③ the granulated granules were premixed with fillers (if any), disintegrants and anti-sticking agents to obtain premixed granules; (4) then the lubricants and the premixed granules were mixed to obtain a total mixed granules; and ⑤ the total mixed granules were filled into suitable hollow capsule shells. Optionally, antioxidants can also be added in step ③.
Method C:
   ① the active ingredient was mixed with fillers, disintegrants, binders (if any) and antioxidants to obtain a premix; ② the premix was subjected to wet granulation and dried to obtain dry granules; ③ the dry granules were premixed with fillers, disintegrants and anti-sticking agents to obtain premixed granules; (4) then the lubricants and the premixed granules were mixed to obtain a total mixed granules; and ⑤ the total mixed granules were filled into suitable hollow capsule shells. Optionally, the binders can also be added after preparing the binder solution in step ②, and/or the antioxidant can also be added in step ③.

Specifically, capsule samples A1 and A2 were prepared according to method A above, samples A3 and A4 were prepared according to method B, and samples A5 and A6 were prepared according to method C. The composition of each sample is shown in Tables A-C below.

**Table A**

| Name of raw and auxiliary material | Function | A1 | | A2 | |
|---|---|---|---|---|---|
| | | Additive Amount (mg) | Proportio n% | Additive Amount (mg) | Proportion % |
| Active ingredient | Active drug | 54.54 | 34.09 | 54.54 | 34.09 |
| Mannitol | Filler | 64.36 | 40.22 | 64.36 | 40.22 |
| Pregelatinized starch | Filler | 32.22 | 20.14 | 32.22 | 20.14 |
| Crospovidone | Disintegrant | 4.00 | 2.50 | 4.00 | 2.50 |
| Colloidal silica | Anti-sticking agent | 1.60 | 1.00 | 1.60 | 1.00 |
| Magnesium stearate | Lubricant | 3.20 | 2.00 | 3.20 | 2.00 |
| Butylated hydroxyanisole | Antioxidant | 0.08 | 0.05 | N/A | N/A |
| Butylated hydroxytoluene | Antioxidant | N/A | N/A | 0.08 | 0.05 |
| Total | N/A | 160.00 | 100.00 | 160.00 | 100.00 |

| | | | | | |
|---|---|---|---|---|---|
| "N/A" stands for "none" in this application. | | | | | |

**Table B**

| Name of raw and auxiliary material | Function | A3 | | A4 | |
|---|---|---|---|---|---|
| | | Additive Amount (mg) | Proportion% | Additive Amount (mg) | Proportion% |
| Active ingredient | Active drug | 54.55 | 21.82 | 54.55 | 21.82 |
| Microcrystalline cellulose | Filler | 109.08 | 43.63 | 109.00 | 43.60 |
| Pregelatinized starch | Filler | 55.00 | 22.00 | 55.00 | 22.00 |
| HPMC | Binder | 5.00 | 2.00 | 5.00 | 2.00 |
| Carboxymethyl starch sodium | Disintegrant | 12.50 | 5.00 | 12.50 | 5.00 |
| Colloidal silica | Anti-sticking agent | 6.25 | 2.50 | 6.25 | 2.50 |
| Magnesium stearate | Lubricant | 7.50 | 3.00 | 7.50 | 3.00 |
| Butylated hydroxyanisole | Antioxidant | 0.12 | 0.05 | 0.12 | 0.05 |
| Butylated hydroxytoluene | Antioxidant | N/A | N/A | 0.08 | 0.03 |
| Total | N/A | 250.00 | 100.00 | 250.00 | 100.00 |

**Table C**

| Name of raw and auxiliary material | Function | A5 | | A6 | |
|---|---|---|---|---|---|
| | | Additive Amount (mg) | Proportion% | Additive Amount (mg) | Proportion% |
| Active ingredient | Active drug | 54.55 | 21.82 | 54.55 | 21.82 |
| Microcrystalline cellulose | Filler | 115.33 | 46.13 | 115.25 | 46.10 |
| Pregelatinized starch | Filler | 55.00 | 22.00 | 55.00 | 22.00 |
| Carboxymethyl starch sodium | Disintegrant | 11.25 | 4.50 | 11.25 | 4.50 |
| Colloidal silica | Anti-sticking agent | 6.25 | 2.50 | 6.25 | 2.50 |
| Magnesium stearate | Lubricant | 7.50 | 3.00 | 7.50 | 3.00 |
| Butylated hydroxyanisole | Antioxidant | 0.12 | 0.05 | 0.12 | 0.05 |
| Butylated hydroxytoluene | Antioxidant | N/A | N/A | 0.08 | 0.03 |
| Total | N/A | 250.00 | 100.00 | 250.00 | 100.00 |

According to the dissolution method (the first method of General Rule 0931 of Chinese Pharmacopoeia 2020 edition), 900 mL, 0.1 M HCl was used as the dissolution medium at 100 rpm, the dissolution rate of the above-mentioned capsule samples was determined in accordance with the method; and the acceptable standard for the dissolution rate is ≥ 80% at 45 min.

Results: The results of the dissolution test showed that the dissolution rate of each sample was greater than 80% at 45 min.

### Example 2

The active ingredient was prepared into tablets and tested for dissolution.

Wherein, preparation method is as follows:
Method one:
   ① the active ingredient was mixed with fillers, disintegrants, anti-sticking agents, binders (if any) and antioxidants to obtain a premix; ② then the lubricants and the premix were mixed to obtain a total mixed powder; (3) the total mixed powder was compressed into tablets to obtain plain tablets; and (4) the plain tablets were coated to obtain coated tablets.
Method two:
   ① the active ingredient was mixed with fillers, disintegrants, anti-sticking agents, binders (if any), lubricants and antioxidants to obtain a premix; ② the premix was subjected to dry granulation to obtain granulated granules; ③ the granulated granules were premixed with fillers (if any), disintegrants and anti-sticking agents to obtain premixed granules; (4) then the lubricants and the premixed granules were mixed to obtain a total mixed granules; ⑤ the total mixed granules were compressed into tablets to obtain plain tablets; and ⑥ the plain tablets were coated to obtain coated tablets. Optionally, antioxidants can also be added in step ③.
Method three:
   ① the active ingredient was mixed with fillers, disintegrants, binders (if any) and antioxidants to obtain a premix; ② the premix was subjected to wet granulation and dried to obtain dry granules; ③ the dry granules were premixed with fillers, disintegrants and anti-sticking agents to obtain premixed granules; (4) then the lubricants (such as purified water) and the premixed granules were mixed to obtain a total mixed granules; ⑤ the total mixed granules were compressed into tablets to obtain plain tablets; and ⑥ the plain tablets were coated to obtain coated tablets. Optionally, the binders can also be added after preparing the binder solution in step ②, and/or the antioxidant can also be added in step ③.

Wherein, the above-mentioned coating material was a film coating premix, and the dosage was 3,0±1.0% of the weight of the plain tablet.

Specifically, tablet samples B1-B2 and B5-B17 were prepared according to the method three above, and samples B3 and B4 were prepared according to the method two. The composition of plain tablet of each sample is shown in Tables D, E and F1-F6 below.

**Table D**

| Name of raw and auxiliary material | Function | B1 | B2 | Proporti on (%) |
|---|---|---|---|---|
| | | Additive Amount (mg) | Additive Amount (mg) | |
| Active ingredient | Active ingredient | 54.54 | 109.08 | 12.12 |
| Butylated hydroxytoluene | Antioxidant | 0.18 | 0.36 | 0.04 |
| Pregelatinized starch | Filler | 42.75 | 85.50 | 9.50 |
| Microcrystalline cellulose | Filler | 291.78 | 583.56 | 64.84 |
| Povidone | Binder | 11.25 | 22.50 | 2.50 |
| Crospovidone | Disintegrant | 27.00 | 54.00 | 6.00 |
| Colloidal silica | Anti-sticking agent | 6.75 | 13.50 | 1.50 |
| Magnesium stearate | Lubricant | 15.75 | 31.50 | 3.50 |
| Total | N/A | 450.00 | 900.00 | 100.00 |

**Table E**

| Name of raw and auxiliary material | Function | B3 | | B4 | |
|---|---|---|---|---|---|
| | | Additive Amount (mg) | Proportio n% | Additive Amount (mg) | Proportion% |
| Active ingredient | Active drug | 54.55 | 21.82 | 54.55 | 21.82 |
| Microcrystalline cellulose | Filler | 109.08 | 43.63 | 109.00 | 43.60 |
| Pregelatinized starch | Filler | 55.00 | 22.00 | 55.00 | 22.00 |
| HPMC | Binder | 5.00 | 2.00 | 5.00 | 2.00 |
| Carboxymethyl starch sodium | Disintegrant | 12.50 | 5.00 | 12.50 | 5.00 |
| Colloidal silica | Anti-sticking agent | 6.25 | 2.50 | 6.25 | 2.50 |
| Magnesium stearate | Lubricant | 7.50 | 3.00 | 7.50 | 3.00 |
| Butylated hydroxyanisole | Antioxidant | 0.12 | 0.05 | 0.12 | 0.05 |
| Butylated hydroxytoluene | Antioxidant | N/A | N/A | 0.08 | 0.03 |
| Total | N/A | 250.00 | 100.00 | 250.00 | 100.00 |

**Table F1**

| Name of raw and auxiliary material | Function | B5 | | B6 | |
|---|---|---|---|---|---|
| | | Additive Amount (mg) | Proportion % | Additive Amount (mg) | Proportion % |
| Active ingredient | Active drug | 54.55 | 21.82 | 54.55 | 21.82 |
| Microcrystalline cellulose | Filler | 115.33 | 46.13 | 115.25 | 46.10 |
| Pregelatinized starch | Filler | 55.00 | 22.00 | 55.00 | 22.00 |
| HPMC | Binder | N/A | N/A | N/A | N/A |
| Carboxymethyl starch sodium | Disintegrant | 11.25 | 4.50 | 11.25 | 4.50 |
| Colloidal silica | Anti-sticking agent | 6.25 | 2.50 | 6.25 | 2.50 |
| Magnesium stearate | Lubricant | 7.50 | 3.00 | 7.50 | 3.00 |
| Butylated hydroxyanisole | Antioxidant | 0.12 | 0.05 | 0.12 | 0.05 |
| Butylated hydroxytoluene | Antioxidant | N/A | N/A | 0.08 | 0.03 |
| Total | N/A | 250.00 | 100.00 | 250.00 | 100.00 |

**Table F2**

| Name of raw and auxiliary material | Function | B7 | | B8 | |
|---|---|---|---|---|---|
| | | Additive Amount (mg) | Proportion% | Additive Amount (mg) | Proportion% |
| Active ingredient | Active ingredient | 109.08 | 12.12 | 109.08 | 12.12 |
| Microcrystalline cellulose | Filler | 588.06 | 65.34 | 583.56 | 64.84 |
| Pregelatinized starch | Filler | 90.00 | 10.00 | 94.50 | 10.50 |
| Crospovidone | Disintegrant | 54.00 | 6.00 | 54.00 | 6.00 |
| Povidone | Binder | 22.50 | 2.50 | 22.50 | 2.50 |
| Butylated hydroxytoluene | Antioxidant | 0.36 | 0.04 | 0.36 | 0.04 |
| Colloidal silica | Anti-sticking agent | 9.00 | 1.00 | 9.00 | 1.00 |
| Magnesium stearate | Lubricant | 27.00 | 3.00 | 27.00 | 3.00 |
| Total | N/A | 900.00 | 100.00 | 900.00 | 100.00 |

**Table F3**

| Name of raw and auxiliary material | Function | B9 | | B10 | |
|---|---|---|---|---|---|
| | | Additive Amount (mg) | Proportion% | Additive Amount (mg) | Proportion% |
| Active ingredient | Active ingredient | 109.08 | 12.12 | 109.08 | 12.12 |
| Microcrystalline cellulose | Filler | 592.56 | 65.84 | 574.56 | 63.84 |
| Pregelatinized starch | Filler | 90.00 | 10.00 | 90.00 | 10.00 |
| Crospovidone | Disintegrant | 54.00 | 6.00 | 72.00 | 8.00 |
| Povidone | Binder | 18.00 | 2.00 | 18.00 | 2.00 |
| Butylated hydroxytoluene | Antioxidant | 0.36 | 0.04 | 0.36 | 0.04 |
| Colloidal silica | Anti-sticking agent | 9.00 | 1.00 | 9.00 | 1.00 |
| Magnesium stearate | Lubricant | 27.00 | 3.00 | 27.00 | 3.00 |
| Total | N/A | 900.00 | 100.00 | 900.00 | 100.00 |

**Table F4**

| Name of raw and auxiliary material | Function | B11 | | B12 | | B13 | |
|---|---|---|---|---|---|---|---|
| | | Additive Amount (mg) | Proporti on% | Additive Amount (mg) | Proporti on% | Additive Amount (mg) | Proporti on% |
| Active ingredient | Active ingredient | 109.08 | 12.12 | 109.08 | 12.12 | 109.08 | 12.12 |
| Microcrystalline cellulose | Filler | 583.56 | 64.84 | 583.56 | 64.84 | 583.56 | 64.84 |
| Pregelatinized starch | Filler | 90.00 | 10.00 | 85.50 | 9.50 | 90.0 | 10.0 |
| Crospovidone | Disintegrant | 54.00 | 6.00 | 54.00 | 6.00 | 54.0 | 6.0 |
| Povidone | Binder | 22.50 | 2.50 | 22.50 | 2.50 | 27.0 | 3.0 |
| Butylated hydroxytoluene | Antioxidant | 0.36 | 0.04 | 0.36 | 0.04 | 0.36 | 0.04 |
| Colloidal silica | Anti-sticking agent | 13.50 | 1.50 | 18.00 | 2.00 | 9.0 | 1.0 |
| Magnesium stearate | Lubricant | 27.00 | 3.00 | 27.00 | 3.00 | 27.0 | 3.0 |
| Total | N/A | 900.00 | 100.00 | 900.00 | 100.00 | 900 | 100 |

**Table F5**

| Name of raw and auxiliary material | Function | B14 | | B15 | |
|---|---|---|---|---|---|
| | | Additive Amount (mg) | Proportion% | Additive Amount (mg) | Proportion% |
| Active ingredient | Active ingredient | 109.08 | 12.12 | 109.08 | 12.12 |
| Microcrystalline cellulose | Filler | 579.06 | 64.34 | 588.06 | 65.34 |
| Pregelatinized starch | Filler | 85.5 | 9.5 | 85.5 | 9.5 |
| Crospovidone | Disintegrant | 54.0 | 6.0 | 54.0 | 6.0 |
| Povidone | Binder | 22.5 | 2.5 | 22.5 | 2.5 |
| Butylated hydroxytoluene | Antioxidant | 0.36 | 0.04 | 0.36 | 0.04 |
| Colloidal silica | Anti-sticking agent | 13.5 | 1.5 | 13.5 | 1.5 |
| Magnesium stearate | Lubricant | 36.0 | 4.0 | 27.0 | 3.0 |
| Total | N/A | 900 | 100 | 900 | 100 |

**Table F6**

| Name of raw and auxiliary material | Function | B16 | | B17 | |
|---|---|---|---|---|---|
| | | Additive Amount (mg) | Proporti on% | Additive Amount (mg) | Proporti on% |
| Active ingredient | Active ingredient | 109.08 | 9.09 | 109.08 | 12.12 |
| Microcrystalline cellulose | Filler | 802.44 | 66.87 | 561.06 | 62.34 |
| Pregelatinized starch | Filler | 114.00 | 9.50 | 81.00 | 9.00 |
| Crospovidone | Disintegrant | 72.00 | 6.00 | 54.00 | 6.00 |
| Povidone | Binder | 30.00 | 2.50 | N/A | N/A |
| Hydroxypropyl methylcellulose | Binder | N/A | N/A | 22.50 | 2.50 |
| Butylated hydroxytoluene | Antioxidant | 0.48 | 0.04 | N/A | N/A |
| Butylated hydroxyanisole | Antioxidant | N/A | N/A | 0.36 | 0.04 |
| Colloidal silica | Anti-sticking agent | 30.00 | 2.50 | 18.00 | 2.00 |
| Magnesium stearate | Lubricant | 42.00 | 3.50 | N/A | N/A |
| Glyceryl behenate | Lubricant | N/A | N/A | 54.00 | 6.00 |
| Total | N/A | 1200.00 | 100.00 | 900.00 | 100.00 |

The dissolution method (the second method of General Rule 0931 of Chinese Pharmacopoeia 2020 edition) was used to determine the dissolution rate of the above-mentioned tablet samples with 900 mL of pH 3.0 potassium hydrogen phthalate buffer solution + 1.5% Tween 80 as the dissolution medium at 75 rpm; and the acceptable standard for the dissolution rate is ≥ 80% at 45 min.

Results: The results of the dissolution test showed that the dissolution rate of each sample was greater than 80% at 45 min, and the dissolution rate of most samples was greater than 90% at 45 min.

### Comparative example

According to the methods for the aforementioned capsules and tablets, without adding antioxidants, the capsule and tablet samples without antioxidants were prepared as comparison samples. Specifically, according to the method B and method C of Example 1, the capsule comparison samples C1 and C2 without antioxidants were respectively prepared; and according to the method two and the method three of Example 2, the tablet comparison samples C3 and C4 without antioxidants were respectively prepared. The specific composition of each comparison sample is shown in Tables G1 and G2 below.

**Table G1 Capsule comparison sample**

| Name of raw and auxiliary material | Function | C1 | | C2 | |
|---|---|---|---|---|---|
| | | Additive Amount (mg) | Proportion% | Additive Amount (mg) | Proportion% |
| Active ingredient | Active drug | 54.55 | 21.82 | 54.55 | 21.82 |
| Microcrystalline cellulose | Filler | 115.45 | 46.18 | 109.20 | 43.68 |
| Pregelatinized starch | Filler | 55.00 | 22.00 | 55.00 | 22.00 |
| HPMC | Binder | N/A | N/A | 5.00 | 2.00 |
| Carboxymethyl starch sodium | Disintegrant | 11.25 | 4.50 | 12.50 | 5.00 |
| Colloidal silica | Anti-sticking agent | 6.25 | 2.50 | 6.25 | 2.50 |
| Magnesium stearate | Lubricant | 7.50 | 3.00 | 7.50 | 3.00 |
| Total | N/A | 250.00 | 100.00 | 250.00 | 100.00 |

**Table G2 Tablet comparison sample**

| Name of raw and auxiliary material | Function | C3 | | C4 | |
|---|---|---|---|---|---|
| | | Additive Amount (mg) | Proportion% | Additive Amount (mg) | Proportion% |
| Active ingredient | Active drug | 54.55 | 21.82 | 54.55 | 21.82 |
| Microcrystalline cellulose | Filler | 115.45 | 46.18 | 109.20 | 43.68 |
| Pregelatinized starch | Filler | 55.00 | 22.00 | 55.00 | 22.00 |
| HPMC | Binder | N/A | N/A | 5.00 | 2.00 |
| Carboxymethyl starch sodium | Disintegrant | 11.25 | 4.50 | 12.50 | 5.00 |
| Colloidal silica | Anti-sticking agent | 6.25 | 2.50 | 6.25 | 2.50 |
| Magnesium stearate | Lubricant | 7.50 | 3.00 | 7.50 | 3.00 |
| Total | N/A | 250.00 | 100.00 | 250.00 | 100.00 |

### Example 3

With reference to the 9001 guidelines for the stability test of raw materials and preparations in "Chinese Pharmacopoeia 2015 Edition IV", the stability of the capsule and tablet products of the present invention and the comparison samples C1-C4 were investigated. The specific experimental method is as described in the present invention, and the experimental results are shown in Table H below.

**Table H**

| Sample number | Investigation project | Time (month) | | | | | |
|---|---|---|---|---|---|---|---|
| | | 0 | 1 | 2 | 3 | 4 | 6 |
| C1 | Total impurity (%) (Acceptance criteria: ≤ 1.5) | 0.28 | 0.36 | 0.51 | 0.91 | 3.38 | Not detected |
| C2 | | 0.27 | 0.40 | 0.75 | 1.97 | 3.21 | Not detected |
| C3 | | 0.27 | 0.32 | 0.49 | 1.77 | 1.55 | Not detected |
| C4 | | 0.27 | 0.33 | 1.12 | 1.87 | 2.25 | Not detected |
| A1 | | 0.26 | 0.26 | 0.28 | 0.35 | 0.40 | 0.41 |
| A2 | | 0.25 | 0.27 | 0.32 | 0.41 | 0.40 | 0.46 |
| A3 | | 0.33 | 0.38 | 0.44 | 0.52 | 0.57 | 0.65 |
| A4 | | 0.27 | 0.35 | 0.44 | 0.51 | 0.56 | 0.67 |
| A5 | | 0.28 | 0.36 | 0.40 | 0.49 | 0.58 | 0.63 |
| A6 | | 0.36 | 0.39 | 0.43 | 0.50 | 0.56 | 0.69 |
| B1 | | 0.05 | 0.05 | 0.07 | 0.06 | Not detected | 0.21 |
| B2 | | 0.05 | 0.06 | 0.06 | 0.06 | Not detected | 0.06 |
| B3 | | 0.27 | 0.33 | 0.27 | 0.35 | 0.42 | 0.45 |
| B4 | | 0.26 | 0.33 | 0.27 | 0.34 | 0.42 | 0.43 |
| B5 | | 0.28 | 0.33 | 0.28 | 0.33 | 0.41 | 0.41 |
| B6 | | 0.26 | 0.35 | 0.31 | 0.35 | 0.41 | 0.40 |
| B16 | | 0.18 | 0.23 | 0.25 | 0.29 | Not detected | 0.40 |
| B17 | | 0.11 | 0.14 | 0.15 | 0.15 | Not detected | 0.15 |

As can be seen from the above table that the total impurity content of the comparison samples increased significantly after 1 month of the experiment, and the total impurity content exceeded the acceptable limit after 3-4 months; it can be seen that the stability of the comparison samples is poor. And the total impurity content of the capsules and tablets of the present invention is substantially unchanged during the experiment, and is significantly lower than the total impurity content of the comparison samples; especially after long-term storage, the total impurity content of each sample had basically no obvious change, and was much lower than that of the comparison samples. It can be seen that when preparing capsules or tablets, adding an antioxidant can reduce the amount and rate of impurities generated during storage and improve the stability of the tablets.

Reference throughout this specification to "an embodiment," "some embodiments," "an example," "a specific example," or "some examples," means that a particular feature, structure, material, or characteristic described in connection with the embodiment or example is included in at least one embodiment or example of the present disclosure. The schematic representation of the above terms throughout this specification are not necessarily referring to the same embodiment or example of the present disclosure. Furthermore, the particular features, structures, materials, or characteristics may be combined in any suitable manner in one or more embodiments or examples. In addition, those skilled in the art can integrate and combine different embodiments, examples or the features of them as long as they are not contradictory to one another.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure.

## Claims

1. A composition **characterized by** comprising 3-(4-(dihexylamino)-3-fluorophenyl)-2,6-dimethylpyrimidin-4(3*H*)-one or a pharmaceutically acceptable salt thereof and an antioxidant.

2. The composition of claim 1, **characterized in that**, the pharmaceutically acceptable salt of the 3-(4-(dihexylamino)-3-fluorophenyl)-2,6-dimethylpyrimidin-4(3*H*)-one is hydrochloride thereof.

3. The composition of any one of claims 1-2, **characterized in that**, the antioxidant is an antioxidant reactive with free radicals.

4. The composition of any one of claims 1-3, **characterized in that**, the antioxidant is butylated hydroxyanisole, butylated hydroxytoluene, propyl gallate or any combination thereof.

5. The composition of any one of claims 1-4, **characterized in that**, the composition is an oral solid formulation;
optionally, the oral solid formulation is a capsule or a tablet.

6. The composition of any one of claims 1-5, **characterized in that**, the composition further comprises pharmaceutically acceptable excipients;
optionally, the excipient includes at least one selected from a filler, a disintegrant, a binder, a lubricant and an anti-sticking agent.

7. The composition of claim 6, **characterized in that**, the filler is optionally selected from at least one of mannitol, pregelatinized starch, microcrystalline cellulose, lactose, starch, calcium hydrogen phosphate, sorbitol, sucrose, lactitol and maltose;
the disintegrant is optionally selected from at least one of crospovidone, carboxymethyl starch sodium, croscarmellose sodium and low-substituted hydroxypropyl cellulose;
the anti-sticking agent is optionally selected from at least one of colloidal silicon dioxide and talc powder;
the lubricant is optionally selected from at least one of magnesium stearate, sodium stearyl fumarate, stearic acid, calcium stearate and glyceryl behenate; and
the binder is optionally selected from at least one of povidone, hydroxypropyl methylcellulose, hydroxypropyl cellulose, polyethylene glycol, ethylcellulose and methylcellulose.

8. The composition of any one of claims 1-7, **characterized in that**, the content of the antioxidant is 0.01~0.40, 0.02~0.40, 0.01~0.30, 0.01~0.20, 0.02~0.30, 0.03~0.30, 0.03~0.20, 0.03~0.10, 0.05~0.20, 0.08~0.30, 0.08~0.20, 0.08~0.10, 0 .04- 0.30, 0.03~0.08, 0.03~0.05, 0.04~0.05 or 0.05~0.08 parts by weight.

9. The composition of any one of claims 1-8, **characterized in that**, the content of the 3-(4-(dihexylamino)-3-fluorophenyl)-2,6-dimethylpyrimidin-4(3*H*)-one hydrochloride is 5.00~73.00, 6.00~58.00, 9.00~58.00, 11.00~58.00, 9.00~35.00, 12.00~35.00, 6.00~22.00, 9.00~22.00, 21.00~35.00 or 12.00~22.00 parts by weight.

10. The composition of any one of claims 6-9, **characterized in that**, the content of the filler is 22.50~91.00, 29.00~82.00, 60.00~77.00, 60.00~76.00, 60.00~69.00, 55.00~90.00, 65.00~77.00, 65.00~76.00 or 70.00~76.00 parts by weight;
optionally, the content of the anti-sticking agent is 0.50~3.50, 0.50~2.50, 0.50~1.50, 1.00~2.50 or 1.00~2.00 parts by weight;
optionally, the content of the disintegrant is 0.50~16.00, 0.05~6.00, 1.50~6.00, 2.50~5.00, 3.00~10.00, 4.00~9.00, 4.00~8.00 or 2.50~8.00 parts by weight;
optionally, the content of the lubricant is 0.50~7.00, 0.50~5.00, 1.00~6.00, 1.00~5.00, 2.00~6.00, 2.00~3.00, 3.00~6.00, 3.00~4.00 or 2.00~4.00 parts by weight; and
optionally, the content of the binder is 0~3.50, 0~3.00, 0~2.00 or 2.00~3.00 parts by weight.

11. The composition of any one of claims 2-10, **characterized in that**, the composition is a capsule, which comprises 3-(4-(dihexylamino)-3-fluorophenyl)-2,6-dimethylpyrimidin-4(377)-one hydrochloride, an antioxidant, a filler, a disintegrant, an anti-sticking agent, a lubricant and optionally a binder; and
optionally, the mass ratio of 3-(4-(dihexylamino)-3-fluorophenyl)-2,6-dimethylpyrimidin-4(3*H*)-one hydrochloride to the antioxidant is (5.00~73.00): (0.01~0.40) or (21.00~35.00): (0.05~0.08).

12. The composition of claim 11, **characterized in that**, the capsule comprises:
5.00~73.00 or 21.00~35.00 parts by weight of 3-(4-(dihexylamino)-3-fluorophenyl)-2,6-dimethylpyrimidin-4(3*H*)-one hydrochloride;
0.01-0.40 or 0.02~0.10 parts by weight of antioxidant;
22.5~90.50 parts by weight of filler;
0.50~6.00 or 2.00~6.00 parts by weight of disintegrant;
0.50~3.50 parts by weight of anti-sticking agent;
0.50~3.50 parts by weight of lubricant; and
0~2.50 parts by weight of binder;

13. The composition of any one of claims 11-12, **characterized in that**, the capsule comprises:
13.00~58.00 or 34.00~34.50 parts by weight of 3-(4-(dihexylamino)-3-fluorophenyl)-2,6-dimethylpyrimidin-4(3*H*)-one hydrochloride;
20.00~55.00 or 40.00~41.00 parts by weight of mannitol;
9.00~28.00 or 20.00~21.00 parts by weight of pregelatinized starch;
1.50~3.50 or 2.00~3.00 parts by weight of crospovidone;
0.50~2.50 or 0.50~1.50 parts by weight of colloidal silicon dioxide; and
0.50~3.00 or 1.50~2.50 parts by weight of magnesium stearate; and
at least one selected from the following:
0.03~0.20 or 0.04~0.06 parts by weight of butylated hydroxyanisole; and
0.03~0.10 or 0.04~0.06 parts by weight of butylated hydroxytoluene.

14. The composition of any one of claims 11-12, **characterized in that**, the capsule comprises:
21.00~22.00 parts by weight of 3-(4-(dihexylamino)-3-fluorophenyl)-2,6-dimethylpyrimidin-4(3*H*)-one hydrochloride;
43.00~47.00 parts by weight of microcrystalline cellulose;
20.00~23.00 parts by weight of pregelatinized starch;
4.00~6.00 or 4.00~5.50 parts by weight of carboxymethyl starch sodium;
0~2.00 parts by weight of hydroxypropyl methylcellulose;
2.00~3.00 parts by weight of colloidal silicon dioxide;
2.00~3.50 or 2.50~3.50 parts by weight of magnesium stearate; and
at least one selected from the following:
0.03~0.10 or 0.04~0.06 parts by weight of butylated hydroxyanisole; and
0.02~0.10 or 0.02~0.05 parts by weight of butylated hydroxytoluene.

15. The composition of any one of claims 2-10, **characterized in that**, the composition is a tablet, which comprises 3-(4-(dihexylamino)-3-fluorophenyl)-2,6-dimethylpyrimidin-4(3*H*)-one hydrochloride, an antioxidant, a filler, a disintegrant, an anti-sticking agent, a lubricant and optionally a binder;
optionally, the mass ratio of 3-(4-(dihexylamino)-3-fluorophenyl)-2,6-dimethylpyrimidin-4(3*H*)-one hydrochloride to the antioxidant is (6.00~22.00): (0.02~0.30) or (9.00~22.00): (0.03~0.10) or (12.00~22.00): (0.03~0.08).

16. The composition of claim 15, **characterized in that**, the tablet comprises:
6.00~22.00, 9.00~22.00 or 12.00~22.00 parts by weight of 3-(4-(dihexylamino)-3-fluorophenyl)-2,6-dimethylpyrimidin-4(3H)-one hydrochloride;
0.02~0.30, 0.03~0.10 or 0.04~0.08 parts by weight of antioxidant;
55.00~90.00, 60.00~76.00, 60.00~77.00 or 65.00~76.00 parts by weight of filler;
0~3.50 parts by weight of binder;
2.00~12.00 or 3.00~10.00 parts by weight of disintegrant;
0.50~3.00 or 1.00~2.50 parts by weight of anti-sticking agent; and
2.50~6.00 or 2.50~5.00 or 3.00~4.00 parts by weight of lubricant.

17. The composition of any one of claims 15-16, **characterized in that**, the tablet comprises:
9.00~13.00 or 11.00~13.00 or 12.00~13.00 parts by weight of 3-(4-(dihexylamino)-3-fluorophenyl)-2,6-dimethylpyrimidin-4(3*H*)-one hydrochloride;
9.00~10.50 or 9.00~10.00 parts by weight of pregelatinized starch;
60.00~70.00 or 62.00~67.00 or 64.00~66.00 parts by weight of microcrystalline cellulose;
1.00~4.00 or 2.00~3.00 parts by weight of povidone or hydroxypropyl methylcellulose;
4.00~12.00, 4.00~9.00, 5.00~9.00 or 6.00~8.00 parts by weight of crospovidone;
0.50~2.50 or 1.00~2.00 parts by weight of colloidal silicon dioxide; and
2.50~4.50 or 3.00~4.00 parts by weight of magnesium stearate; or 5.00~7.00 parts by weight of glyceryl behenate; and
at least one selected from the following:
0.03~0.05 or 0.03~0.04 parts by weight of butylated hydroxytoluene; and
0.03~0.08 or 0.03~0.05 parts by weight of butylated hydroxyanisole.

18. The composition of any one of claims 15-16, **characterized in that**, the tablet comprises:
21.00~22.00 parts by weight of 3-(4-(dihexylamino)-3-fluorophenyl)-2,6-dimethylpyrimidin-4(3*H*)-one hydrochloride;
20.00~25.00 or 23.00~24.00 parts by weight of pregelatinized starch;
40.00~50.00 or 43.00~47.00 parts by weight of microcrystalline cellulose;
0~3.00 or 0~2.00 parts by weight of hydroxypropyl methylcellulose;
4.00~5.50 or 4.50~5.00 parts by weight of carboxymethyl starch sodium;
2.00~3.00 parts by weight of colloidal silicon dioxide; and
2.00~4.00 or 2.50~3.50 parts by weight of magnesium stearate; and
at least one selected from the following:
0.04~0.08 or 0.04~0.06 parts by weight of butylated hydroxyanisole; and
0.02~0.05 or 0.02~0.04 parts by weight of butylated hydroxytoluene.

19. The composition of any one of claims 15-18, **characterized in that**, the tablet further comprises a coating material;
optionally, the weight of the coating material is 2.0-4.0% of the weight of the plain tablet of the tablet.
